# EUROPÄISCHE PATENTSCHRIFT

(11) **EP 0 367 019 B1**
(45) Veröffentlichungstag und Bekanntmachung des Hinweises auf die Patenterteilung: **12.05.1993**
(21) Anmeldenummer: 89119301.3
(22) Anmeldetag: 18.10.1989
(51) Int. Cl.: C07C 237/08, C07C 235/10, C07C 237/02, C07C 235/40, C07C 235/28, C07C 235/26, C07D 295/185, C07D 295/125

(54) **Arzneimittel enthaltend Nitroxyalkylamine mit Amidfunktion und Verfahren zur Herstellung dieser Verbindungen**
Pharmaceutical compositions containing nitroxyalkyl amine with an amide function, and method for the preparation of the compounds
Compositions pharmaceutiques qui contienent des nitroxyamines avec des groupes amides, et procédé pour la préparation des composés

(30) Priorität: 22.10.1988 DE 3836021
(43) Veröffentlichungstag der Anmeldung: 09.05.1990
(73) Patentinhaber: BOEHRINGER MANNHEIM GMBH, 68298 Mannheim (DE)
(72) Erfinder: Simon, Herbert, Dr.rer.nat., D-6840 Lampertheim (DE); Michel, Helmut, D-6800 Mannheim 31 (DE); Schultz, Michael, Dr.rer.nat., D-6800 Mannheim 1 (DE); Bartsch, Wolfgang, Dr.med.vet., D-6806 Viernheim (DE)

(56) Entgegenhaltungen:
- EP-A- 0 090 063
- EP-A- 0 200 915
- EP-A- 0 280 951
- GB-A- 961 317
- GB-A- 1 602 412
- US-A- 2 991 290
- US-A- 3 189 646
- US-A- 4 306 080

## Beschreibung

Gegenstand der vorliegenden Erfindung sind neue Arzneimittel, die als Wirkstoffe Nitroxyalkylamin-Derivate der allgemeinen Formel I 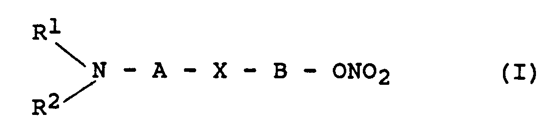
enthalten, worin
- R¹: Wasserstoff, eine geradkettige oder verzweigte, gesättigte oder ungesättigte C₁-C₈-Alkylgruppe, eine C₃-C₈-Cycloalkyl- oder C₈-Cycloalkenylgruppe, eine Aminocarbonyl-, C₁-C₆-Alkylaminocarbonyl- oder Di-C₁-C₆-alkyl-aminocarbonylgruppe bedeutet,
- R²: Wasserstoff, eine geradkettige oder verzweigte, gesättigte oder ungesättigte C₁-C₆-Alkylgruppe, oder eine C₃-C₈-Cycloalkyl-oder C₈ Cycloalkenylgruppe bedeutet, oder R² zusammen mit R¹ und dem Stickstoffatom, an das sie gebunden sind, einen heteroaliphatischen Ring mit bis zu 6 C-Atomen bilden, worin ein oder zwei C-Atome auch durch Stickstoff- oder Sauerstoffatome ersetzt sein können,
- A: einen Valenzstrich oder eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
- B: eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
- X: eine -NR³-CO-Gruppe oder -CO-NR³-Gruppe bedeutet,
wobei
- R³: Wasserstoff oder eine gesättigte oder ungesättigte Alkyl-Gruppe von 1-6 C-Atomen bedeutet, oder für den Fall, daß X die Gruppe -NR³-CO- darstellt, R³ gemeinsam mit den Stickstoffatom und einem C-Atom der Gruppe A einen heterocyclischen Ring mit 4 bis 6 C-Atomen aufspannt, oder R³ gemeinsam mit R², A und den beiden Stickstoffatomen einen heterocyclischen Ring mit 3 bis 5 C-Atomen aufspannt
oder deren optisch aktiven Formen sowie physiologisch verträglichen Salze.

Verbindungen, in denen X die Gruppe -NH-CO- und R¹ und R² Wasserstoff bedeuten, sind bereits als Zwischenprodukte beschrieben in EP-A-0 200 915 (vgl. dort Verbindungen der allgemeinen Formel IV). Für den Fall, daß X die Gruppe -CO-NR₃- und R₁ und R₂ Wasserstoff darstellen, so sind solche Verbindungen als Zwischenprodukte aus EP-A-0 280 951 (vgl. dort Verbindungen der allgemeinen Formel IV) bekannt. Eine pharmakologische Wirkung ist dort jedoch nicht beschreiben. Alle weiteren Verbindungen sind neu.

Gegenstand der vorliegenden Erfindung sind demnach auch neue Verbindungen der allgemeinen Formel I 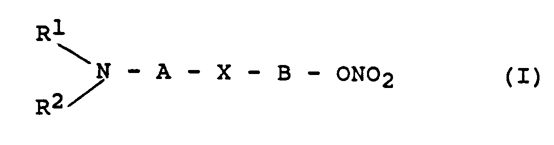
in der
- R¹: Wasserstoff, eine geradkettige oder verzweigte, gesättigte oder ungesättigte C₁-C₈-Alkylgruppe, eine C₃-C₈-Cycloalkyl- oder C₈-Cycloalkenylgruppe, eine Aminocarbonyl-, C₁-C₆-Alkylaminocarbonyl- oder Di-C₁-C₆-alkyl-aminocarbonylgruppe bedeutet,
- R²: Wasserstoff, eine geradkettige oder verzweigte, gesättigte oder ungesättigte C₁-C₆-Alkylgruppe, oder eine C₃-C₈-Cycloalkyl- oder C₈ Cycloalkenylgruppe bedeutet, oder R² zusammen mit R¹ und dem Stickstoffatom, an das sie gebunden sind, einen heteroaliphatischen Ring mit bis zu 6 C-Atomen bilden, worin ein oder zwei C-Atome auch durch Stickstoff- oder Sauerstoffatome ersetzt sein können,
- A: einen Valenzstrich oder eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
- B: eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
- X: eine -NR³-CO-Gruppe oder -CO-NR³-Gruppe bedeutet,
wobei
- R³: Wasserstoff oder eine gesättigte oder ungesättigte Alkyl-Gruppe von 1-6 C-Atomen bedeutet, oder für den Fall, daß X die Gruppe -NR³-CO- darstellt, R³ gemeinsam mit dem Stickstoffatom und einem C-Atom der Gruppe A einen heterocyclischen Ring mit 4 bis 6 C-Atomen aufspannt, oder R³ gemeinsam mit R², A und den beiden Stickstoffatomen einen heterocyclischen Ring mit 3 bis 5 C-Atomen aufspannt,
mit der Maßgabe, daß R¹ und R² nicht gleichzeitig Wasserstoff bedeuten, wenn X die Gruppe -NH-CO- oder -CO-NR₃- ist, sowie deren optisch aktive Formen und physiologisch verträgliche Salze.

Die erfindungsgemäßen Arzneimittel enthaltend Verbindungen der allgemeinen Formel I besitzen wertvolle pharmakologische Eigenschaften. Sie bewirken eine Verminderung des Sauerstoffbedarfs des Herzens, eine Erhöhung des Blutflusses und eine Erniedrigung des Blutdrucks. Überraschenderweise wurde nun gefunden, daß die beanspruchten Verbindungen eine nitratartige Wirkung von besonders langer Wirkdauer aufweisen. Sie eignen sich daher zur Prophylaxe oder Behandlung von Herz- und Kreislauferkrankungen, wie z.B. Angina pectoris.

R¹ kann Wasserstoff oder eine geradkettige, verzweigte oder cyclische Alkylkette von 1-8 C-Atomen, bevorzugt Methyl, Ethyl, n-Propyl, Isopropyl, n-Butyl, n-Pentyl, Cyclopropyl, Cyclopentyl, Cyclohexyl und Cycloheptyl bedeuten, ferner kommen z.B. Isobutyl, 2-Pentyl, 2-Hexyl, n-Heptyl, n-Octyl, Cyclobutyl und Cyclooctyl in Frage. R¹ kann auch eine Alkylaminocarbonylgruppe bedeuten, wobei bevorzugt die Methylaminocarbonyl-, Ethylaminocarbonyl-, iso-Propylaminocarbonyl- oder tert.-Butylaminocarbonylgruppe in Frage kommt.

R² kann Wasserstoff oder eine geradkettige, verzweigte oder cyclische Alkylkette von 1-6 C-Atomen, bevorzugt Methyl, Ethyl, n-Propyl, Isopropyl, n-Butyl, n-Pentyl, n-Hexyl, Cyclopentyl oder Cyclohexyl bedeuten, ferner kommen z.B. 2-Butyl, Isobutyl, 2-Pentyl, 2-Hexyl oder Cyclopropyl in Frage. Darüberhinaus kann R² gemeinsam mit R¹ und dem benachbarten Stickstoff-Atom einen heteroaliphatischen Ring von 2-6 C-Atomen bilden, wobei 1 oder 2 C-Atome auch durch Stickstoff- oder Sauerstoff-Atome ersetzt sein können. Als Beispiele sind der Aziridin-Ring, der Azetidin-Ring, der Pyrrolidin-Ring, der Piperidin-Ring, der Perhydroazepin-Ring, der Piperazin und der Morpholin-Ring zu nennen, wobei insbesondere der Pyrrolidin-, Piperidin- und Morpholinring bevorzugt sind.

A kann eine geradkettige Alkylenkette von 1-8, bevorzugt 1-5 C-Atomen, bevorzugt Methylen, Ethylen, Trimethylen, Tetramethylen, Pentamethylen, ferner Hexamethylen, Heptamethylen oder Octamethylen bedeuten. Die Alkylenkette A kann auch verzweigt sein und aus 2-8, bevorzugt 2-5 C-Atomen bestehen. Bevorzugt sind z.B. Methylmethylen, 1-Methylethylen, Dimethylmethylen, 1,1-Dimethylethylen, 1,1-Dimethyl-trimethylen, 1-Methyl-tri-methylen; ferner kommen z.B. 1,1-Dimethylmethylen, 1-Ethyl-methylen, 1,1-Dimethyl-tetramethylen, 1,2-Dimethyl-trimethylen, 2,2-Dimethyl-trimethylen, 3,3-Dimethyl-trimethylen, 1,1-Dimethyl-pentamethylen, 1,1-Dimethyl-hexamethylen, 1-Methyl-tetramethylen in Frage. Eine -CH₂-Gruppe oder Alkylengruppe kann durch eine Cycloalkylen-Gruppe mit 3-7, vorzugsweise 6 C-Atomen ersetzt sein. Vorzugsweise kommen der 1,2-, 1,3- und 1,4-Cyclohexylen-Rest in Frage, wobei die Stellung der Substituenten jeweils cis oder trans sein kann. Ferner kann z.B. die Methylen-1,2-, -1,3- oder -1,4-cyclohexylen-Gruppe, die Methylen-1,2-, -1,3- oder -1,4-cyclohexylen-methylen-Gruppe, die Ethylen-1,2-, -1,3- oder -1,4-cyclohexylen-Gruppe, die Trimethylen-1,2-, -1,3- oder -1,4-cyclohexylen-Gruppe, die Tetramethylen-1,2-, -1,3- oder -1,4-cyclohexylen-Gruppe oder auch die Methylen-1,2-, -1,3- oder 1,4-cyclohexylen-ethylen-Gruppe oder die Ethylen-1,2-, -1,3- oder -1,4-cyclohexylen-tetramethylen-Gruppe eingesetzt werden, die Konfiguration der Cycloalkylen-Gruppe kann jeweils cis oder trans sein. Weitere Alkylenketten A sind z.B. die 1,2-Cyclopropylen-Gruppe, die Methylen-1,2-cyclopropylen-Gruppe, die Tetramethylen-1,2-cyclopropylen-Gruppe, die Methylen-1,2-cyclopropylen-methylen-Gruppe, die Methylen-1,2-cyclopropylen-ethylen-Gruppe, die 1,2- oder 1,3-Cyclobutylen-Gruppe, die Methylen-1,2- oder -1,3-cyclobutylen-Gruppe, die Tetramethylen-1,2- oder -1,3-cyclobutylen-Gruppe, die Methylen-1,2- oder -1,3-cyclobutylen-methylen-Gruppe, die Methylen-1,2- oder -1,3-cyclobutylen-ethylen-Gruppe, die 1,2- oder 1,3-Cyclopentylen-Gruppe, die Methylen-1,2- oder -1,3-cyclopentylen-Gruppe, die Tetramethylen-1,2- oder -1,3-cyclopentylen-Gruppe, die Methylen-1,2- oder -1,3-cyclopentylen-methylen-Gruppe, die Methylen-1,2- oder -1,3-cyclopentylen-ethylen-Gruppe, die 1,2-, 1,3- oder 1,4-Cycloheptylen-Gruppe, die Methylen-1,2-, -1,3- oder -1,4-cycloheptylen-Gruppe, die Tetramethylen-1,2-, -1,3- oder -1,4-cycloheptylen-Gruppe, die Methylen-1,2-, -1,3- oder -1,4-cycloheptylen-methylen-Gruppe, die Methylen-1,2-, -1,3- oder -1,4-cycloheptylen-ethylen-Gruppe, die Konfiguration der Cycloalkylen-Gruppe kann jeweils cis oder trans sein.

Die Gruppe B kann eine geradkettige oder verzweigte Alkylenkette von 1-8 C-Atomen, vorzugsweise 2-6 C-Atomen, bedeuten, vorzugsweise Ethylen, 1-Methyl-ethylen, 2-Methyl-ethylen, Trimethylen, 1-Methyl-trimethylen, 3-Methyl-trimethylen, Tetramethylen, Pentamethylen, 1-Methyl-tetramethylen, 1-Ethyl-trimethylen, Hexamethylen, 1-Methyl-pentamethylen, 1-Ethyl-tetramethylen, 1-n-Propyl-tri-methylen, 1,1-Dimethyl-ethylen, ferner z.B. Dimethyl-methylen, 1,1-Dimethyl-tri-methylen, 2,2-Dimethyl-trimethylen, 2,2-Dimethyl-tetramethylen oder 1,1-Dimethyl-hexamethylen. Für den Fall, daß eine -CH₂-Gruppe der Kette B durch eine Cycloalkylen-Gruppe von 3-7 C-Atomen ersetzt ist, kommen z.B. die Cyclopentylen-Gruppe, die 1,2-, 1,3- oder 1,4-Cyclohexylen-Gruppe, die Methylen-1,2-cyclohexylen-Gruppe, die Methylen-1,3-cyclohexylen-Gruppe oder die Methylen-1,4-cyclohexylen-Gruppe in Frage, wobei die Konfiguration der Cycloalkylen-Gruppe jeweils cis oder trans sein kann.

R³ kann Wasserstoff oder einen geradkettigen oder verzweigten, gesättigten oder ungesättigten Alkylrest von 1-6, insbesondere 1-3 C-Atomen bedeuten, vorzugsweise die Methyl-Gruppe, die Ethyl-Gruppe, die n-Propyl-Gruppe, die Isopropyl-Gruppe oder die Allyl-Gruppe, ferner z.B. die n-Butyl-Gruppe oder die i-Butyl-Gruppe.

Für den Fall, daß X die Gruppe -NR₃-CO- darstellt und R₃ gemeinsam mit dem Stickstoffatom und einem C-Atom der Gruppe A einen heterocyclischen Ring bildet, so kommt in diesem Sinne bevorzugt ein Pyrrolidin- oder Piperidin-Ring in Frage. Wenn R₃ gemeinsam mit R₂, A und den beiden Stickstoffatomen einen heterocyclischen Ring bildet, dann ist der Piperazinring besonders bevorzugt.

Bevorzugte Verbindungen der allgemeinen Formel I sind solche, in denen
- R₁: ein Wasserstoffatom, eine geradkettige oder verzweigte C₁-C₃-Alkylgruppe, wie z. B. eine Methyl-, Ethyl- oder iso-Propylgruppe, eine Aminocarbonylgruppe, oder eine C₁-C₃-Alkylaminocarbonylgruppe, wie z. B. iso-Propylaminocarbonyl, bedeutet,
- R₂: ein Wasserstoffatom oder eine geradkettige C₁-C₃-Alkylgruppe, wie z. B. Methyl oder Ethyl, bedeutet, oder R₂ zusammen mit R₁ und dem Stickstoffatom einen Pyrrolidin-, Piperidin- oder Morpholinring bildet,
- A: eine geradkettige oder verzweigte Alkylenkette mit 1 - 5 C-Atomen bedeutet, beispielsweise Methylen, Ethylen, Trimethylen, Methylmethylen, Dimethylmethylen, 1,1-Dimethyl-ethylen, 1,1-Dimethyl-trimethylen oder 2,2-Dimethyl-trimethylen,
- B: eine geradkettige oder verzweigte Alkylenkette mit 1 - 6 C-Atomen darstellt, wobei eine CH₂-Gruppe durch Cyclohexylen ersetzt sein kann, beispielsweise Methylen, Ethylen, Trimethylen, Tetramethylen, Pentamethylen, Dimethyl-methylen, 1,1-Dimethyl-ethylen, 2-Methyl-ethylen, 2,2-Dimethyltrimethylen, 1-Methyl-trimethylen, 4-Methyl-tetramethylen, Cyclohexylen, Methylcyclohexylen, Ethyl-cyclohexylen oder Cyclohexyl-methylen,
- X: eine -NR₃-CO oder -CO-NR₃-Gruppe darstellt, wobei
- R₃: ein Wasserstoffatom, oder für den Fall, daß X eine -NR₃-CO-Gruppe darstellt, R₃ gemeinsam mit dem Stickstoffatom und der Gruppe A einen Piperidinring bildet, oder R₃ gemeinsam mit den beiden Stickstoffatomen der Kette A und R₂ einen Piperazinring darstellt.

Die erfindungsgemäßen Arzneimittel werden üblicherweise in Mengen von 20-500 mg Wirkstoff pro Tag, bezogen auf 75 kg Körpergewicht, appliziert. Bevorzugt ist es 1-2 mal pro Tag 1-2 Tabletten mit einem Wirkstoffgehalt von 10-300 mg zu verabreichen. Die Tabletten können auch retardiert sein, wodurch nur noch 1 mal pro Tag 1-2 Tabletten mit 20-600 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Die Verbindungen der allgemeinen Formel I können in an sich bekannter Weise dadurch hergestellt werden, daß man
1) eine Verbindung der allgemeinen Formel II 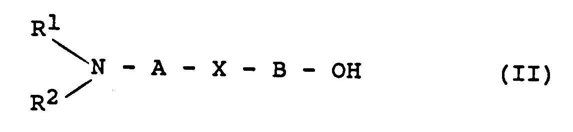 in der R¹, R², A, X und B die oben angegebenen Bedeutungen haben, einer Nitratester-Bildungsreaktion unterwirft, oder,
2.1) für den Fall, daß X eine -NR₃-CO-Gruppe bedeutet, eine Verbindung der allgemeinen Formel III 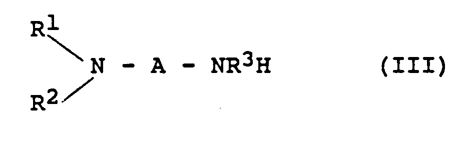 mit einer Verbindung der allgemeinen Formel IV

   Z - CO - B - ONO₂ (IV)

   wobei R¹, R², A, R³ und B die oben angegebenen Bedeutungen haben und Z eine nucleofuge Gruppe bedeutet, umsetzt, beziehungsweise
2.2) für den Fall, daß X eine -CO-NR₃-Gruppe darstellt, eine Verbindung der allgemeinen Formel V

   HNR³ - B - ONO₂ (V)

   mit einer Verbindung der allgemeinen Formel VI 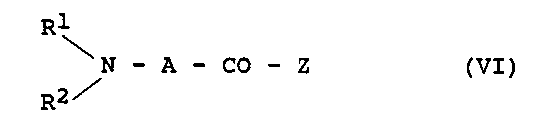 wobei R¹, R², A, R³, B und Z die oben angebenen Bedeutungen haben, umsetzt, oder
2.3) eine Verbindung der allgemeinen Formel VII

   R² - NH - A - X - B - ONO₂ (VII)

   mit Isocyanaten R¹-N=C=O oder Halogenameisensäure-Derivaten R¹-NH-CO-Hal umsetzt, wobei R¹, R², A, X, und B die oben angegebenen Bedeutungen haben und Hal Chlor oder Brom bedeutet.

Verbindungen der allgemeinen Formel II, in denen X die Gruppe -NH-CO- und R¹ und R² Wasserstoff bedeuten, sind bereits aus EP-A-200,915 (vgl. dort Verbindungen der Formel XIV) bekannt. Für den Fall, daß X die Gruppe -CO-NR₃- und R¹ und R² Wasserstoff bedeuten, so sind solche Verbindungen in EP-A-280,951 (vgl. dort Verbindungen der allgemeinen Formel XIII) beschrieben.

Einige Verbindungen der allgemeinen Formel II, in der R₁ und R₂ nicht Wasserstoff bedeuten, sind literaturbekannt und können nach den dort beschriebenen Verfahren hergestellt werden (vgl. hierzu GB-A-961,317; US-A-4,306,080; US-A-3,189,646; US-A-2,991,290; GB-A-1,602,412 oder EP-A-90,063).

Verbindungen der Formel II, in der B eine Cycloalkylengruppe darstellt, sind jedoch neu und ebenfalls Gegenstand der vorliegenden Erfindung.

Ferner sind Verbindungen der allgemeinen Formel II, in der R¹ und R² Wasserstoff bedeuten, als Zwischenprodukte aus EP-A-0 200 915 und EP-A-0 280 951 bekannt. Weiterhin sind einige dieser Verbindungen bereits literaturbekannt: 2-Amino-N-(2-hydroxy-ethyl)-essigsäureamidoxalat aus Helv. Chim. Acta 38 (1955), 1342 und 3-Amino-N-(2-hydroxy-ethyl)-propionsäureamid aus J. Chem. Soc. 1954, 2803.

Die Verbindungen der allgemeinen Formel II können hergestellt werden, indem man in an sich bekannter Weise
1) für den Fall, daß X eine -NR₃-CO-Gruppe bedeutet,
   1.1) eine Verbindung der allgemeinen Formel III mit einer Verbindung der allgemeinen Formel VIII

      Z - CO - B - OH (VIII)

      wobei R¹, R², A, R³, B, und Z die oben angegebenen Bedeutungen haben, umsetzt, oder
   1.2) eine Verbindung der allgemeinen Formel III mit einer Verbindung der allgemeinen Formel IX

      Z - CO - B - O - W (IX)

      wobei R¹, R², A, R³, B und Z die oben angegebenen Bedeutungen haben und W eine Schutzgruppe bedeutet, umsetzt und anschließend die Schutzgruppe W abspaltet, oder
   1.3) eine Verbindung der allgemeinen Formel III mit einem Lacton der allgemeinen Formel X 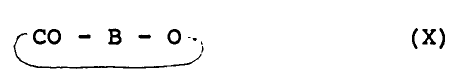 wobei R¹, R², A, R³ und B die oben angegebenen Bedeutungen haben, umsetzt,
2) oder für den Fall, daß X eine -CO-NR₃-Gruppe darstellt,
   2.1) eine Verbindung der allgemeinen Formel VI mit einer Verbindung der allgemeinen Formel XI

      HNR³ - B - OH (XI)

      wobei R¹, R², A, Z, R³ und B die oben angegebenen Bedeutungen haben, umsetzt, oder
   2.2) eine Verbindung der allgemeinen Formel VI mit einer Verbindung der allgemeinen Formel XII

      HNR³ - B - O - W (XII)

      wobei R¹, R², A, Z, R³, B und W die oben genannten Bedeutungen haben, umsetzt, und anschließend die Schutzgruppe W abspaltet, oder
   2.3) eine Verbindung der allgemeinen Formel XIII

      V - A - CO - Z (XIII)

      mit einer Verbindung der allgemeinen Formel XI, wobei A, Z, R³ und B die oben genannten Bedeutungen haben, und V eine Gruppe ist, die in die R¹R²N-Gruppe überführt werden kann, umsetzt, und anschließend die Gruppe V in die R¹R²N-Gruppe überführt, oder
   2.4) eine Verbindung der allgemeinen Formel XIII mit einer Verbindung der allgemeinen Formel XII, wobei V, A, Z, R³, B und W die oben genannten Bedeutungen haben, umsetzt, danach die Gruppe V in die R¹R²N-Gruppe überführt und die Schutzgruppe W abspaltet.

Die Nitratester-Bildungsreaktion der Verbindungen der allgemeinen Formeln II, VIII oder XI kann durchgeführt werden, indem die Verbindungen der allgemeinen Formel II, VIII oder XI mit einem nitratesterbildenden Reagenz, wie rauchender Salpetersäure, einer Mischung aus rauchender Salpetersäure und Acetanhydrid oder einer Mischung aus rauchender Salpetersäure und konz. Schwefelsäure oder Distickstoffpentoxid bei niedrigen Temperaturen in Gegenwart oder Abwesenheit eines inerten Lösungsmittels umsetzt.
Die Reaktionstemperaturen liegen zwischen Raumtemperatur und -60° C, bevorzugt zwischen -10° C und -30° C. Das Molverhältnis der Reaktionspartner liegt zwischen 1 und 10.

Alternativ kann die Nitratester-Bildungsreaktion durchgeführt werden, indem man in einer Verbindung der allgemeinen Formeln II, VIII oder XI selektiv die aliphatische Hydroxyl-Gruppe durch eine Halogen-Gruppe oder Alkyl- oder Arylsulfonsäureester-Gruppe ersetzt und anschließend das Reaktionsprodukt mit z.B. Silbernitrat oder Tetrabutylammoniumnitrat in Gegenwart oder Abwesenheit eines Lösungsmittels bei Temperaturen zwischen Raumtemperatur und 100°C umsetzt. Das Molverhältnis der Umsetzungsreaktion zwischen der Halogenverbindung und Silbernitrat kann zwischen 1 und 10 liegen.

Als nucleofuge Gruppe Z kommen Alkoholate, Halogenide oder Alkylcarboxylate in Frage. Die entsprechenden aktivierten Carbonsäuren IV, VI, VIII, IX oder XIII liegen daher in Form von Estern, Carbonsäurehalogeniden oder -anhydriden vor. Die Aktivierung der Carbonsäuren kann aber auch durch aktivierende Reagenzien wie N,N′-Carbonyldiimidazol, N,N′-Dicyclohexylcarbodiimid, 1-Ethyl-3-(3-dimethylaminopropyl)carbodiimid, 1-Alkyl-2-halogen-pyridiniumsalzen o.ä. Die molaren Verhältnisse zwischen den Reaktionspartnern können zwischen 1 und 100 gewählt werden.

Als Schutzgruppe W wird vorwiegend die Acetyl-Gruppe verwendet. Daneben findet aber auch die Tetrahydropyranyl-, die Benzyl- oder die Benzoyl-Gruppe Verwendung. Die Abspaltung erfolgt gewöhnlich in saurer oder basischer, wäßriger oder alkoholischer Lösung.

Als Schutzgruppe V kommen z.B. die -NO₂-Gruppe, die Azido-Gruppe, die Benzyl- oder Benzyloxycarbonyl-Gruppe oder eine durch 1 oder 2 Benzylreste geschützte Amin-Gruppe in Frage. Außerdem kann V eine nucleofuge Gruppe wie z.B. Halogenide, Mesylat oder Tosylat bedeuten, die mit Benzylgruppen-geschützten primären und sekundären Aminen umgesetzt werden oder den Aminen R¹R²NH umgesetzt werden. Zur Reduktion der -NO₂-Gruppe können zahlreiche Verfahren, z.B. mit Zink in Salzsäure, mit Eisen in Salzsäure, mit Lithiumaluminiumhydrid in Ethern, mit anorganischen Sulfiden wie NaHS, (NH₄)₂S oder Na₂S₂O₄ in wäßriger und/oder alkoholischer Lösung, oder hydrogenolytisch mit Katalysatoren wie PtO₂, Pd oder Raney-Nickel, vorzugsweise in einem alkoholischen Lösungsmittel wie Methanol oder Ethanol, bei Drucken zwischen 1 und 200 bar, angewendet werden. Die Temperaturen können zwischen -20° C und 200° C, vorzugsweise zwischen Raumtemperatur und 100° C liegen. Die Umsetzung von Aminen mit Halogeniden, Mesylaten oder Tosylaten erfolgt in Gegenwart oder Abwesenheit eines Lösungsmitels zwischen 0° C und 150° C, bevorzugt zwischen 20° C und 80° C. Als Lösungsmittel können z.B. Methanol, Ethanol, Propanol, i-Propanol, Ether wie Diethylether, Diisopropylether, tert.-Butylmethylether oder Tetrahydrofuran oder aromatische Lösungsmittel wie Toluol oder Xylol verwendet werden. Das molare Verhältnis der Reaktionspartnerist nicht kritisch. Es können Verhältnisse zwischen 1 und 100 gewählt werden. Gegebenenfalls können die Reaktionen unter erhöhtem Druck durchgeführt werden. Die Abspaltung von Benzyl-Schutzgruppen erfolgt hydrogenolytisch in Gegenwart eines organischen Lösungsmittels und/oder Wasser sowie Palladium auf Kohle als Katalysator. Die Temperaturen können zwischen Raumtemperatur und 250° C, vorzugsweise bei Raumtemperatur und 60° C liegen, der Wasserstoffdruck kann zwischen 1 und 300 bar, vorzugsweise 1 bis 5 bar, betragen.

Die Verbindungen der allgemeinen Formeln I und II können asymmetrische Kohlenstoff-Atome besitzen. Gegenstand der Erfindung sind daher auch sämtliche möglichen Racemate und Diastereomerengemische sowie sämtliche optisch aktiven Formen der erfindungsgemäßen Verbindungen der allgemeinen Formeln I und II. Chirale Verbindungen können entweder durch direkte Synthese oder übliche Racematentrennung erhalten werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Salpetersäure, Phosphorsäure, Schwefelsäure, Ameisensäure, Essigsäure, Propionsäure, Oxalsäure, Fumarsäure, Maleinsäure, Bernsteinsäure, Adipinsäure, Benzoesäure, Salicylsäure, o-Acetoxybenzoesäure, Zimtsäure, Naphthoesäure, Mandelsäure, Citronensäure, Äpfelsäure, Weinsäure, Asparaginsäure, Glutaminsäure, Methansulfonsäure, p-Toluolsulfonsäure oder N-Cyclohexylaminsulfonsäure um.

Die Substanzen der allgemeinen Formeln I und ihre Salze können in flüssiger und fester Form enteral oder parenteral appliziert werden. Als Injektionsmedium, kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- oder Citratpuffer, Ethanol, Komplexbildner (wie Ethylendiamintetraessigsäure oder deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole). Für orale Applikation geeignete Zübereitungen können gewünschtenfalls Geschmacks- und Süßstoffe enthalten.

Neben den nachfolgend aufgeführten Beispielen kommen auch die folgenden Verbindungen beispielhaft im Sinne der vorliegenden Erfindung in Frage:
2-Amino-N-(2-methyl-5-nitroxy-pent-2-yl)-essigsäureamid
2-Dimethylamino-N-(2-methyl-4-nitroxy-but-2-yl)-essigsäureamid
L-2-Amino-N-(2-nitroxy-ethyl)-propionsäureamid
L-2-Amino-N-(2-methyl-1-nitroxy-prop-2-yl)-propionsäureamid
L-2-Amino-N-(3-nitroxy-propyl)-propionsäureamid
2-Amino-N-(2-methyl-1-nitroxy-prop-2-yl)-propionsäureamid
2-Amino-2-methyl-N-ethyl-N-(2-nitroxy-ethyl)-propionsäureamid
2-Methylaminocarbonylamino-2-methyl-N-(2-nitroxy-ethyl)-propionsäureamid
2-Aminocarbonylamino-2-methyl-N-(3-nitroxy-propyl)-propionsäureamid
2-Methylaminocarbonylamino-2-methyl-N-(3-nitroxy-propyl)-propionsäureamid
2-[(2-Methyl-ethyl)aminocarbonylamino]-2-methyl-N-(3-nitroxy-propyl)-propionsäureamid
3-Amino-N-(2-methyl-1-nitroxy-prop-2-yl)-propionsäureamid
3-Amino-N-(1-nitroxy-but-3-yl)-propionsäureamid
3-Amino-N-ethyl-N-(3-nitroxy-propyl)-propionsäureamid
3-Dimethylamino-N-(2-methyl-1-nitroxy-prop-2-yl)-propionsäureamid
3-Dimethylamino-N-(3-methyl-1-nitroxy-but-3-yl)-propionsäureamid
3-Amino-3-methyl-N-(3-nitroxy-propyl)-buttersäureamid
4-Amino-N-(2-methyl-1-nitroxy-prop-2-yl)-buttersäureamid
4-Amino-4-methyl-N-(2-methyl-1-nitroxy-prop-2-yl)-valeriansäureamid
4-Amino-4-methyl-N-(3-methyl-1-nitroxy-but-3-yl)-valeriansäureamid
N-(2-Amino-ethyl)-2-nitroxy-propionsäureamid
N-(2-Amino-ethyl)-3,3-dimethyl-5-nitroxy-valeriansäureamid
N-(2-Aminocarbonylamino-ethyl)-4-nitroxy-buttersäureamid
N-(2-Amino-2-methyl-propyl)-2-nitroxy-propionsäureamid
N-(2-Dimethylamino-ethyl)-2-nitroxy-propionsäureamid
N-(2-Dimethylamino-ethyl)-2-methyl-2-nitroxy-propionsäureamid
N-(2-Dimethylamino-ethyl)-2,2-dimethyl-3-nitroxy-propionsäureamid
N-(2-Dimethylamino-ethyl)-3-nitroxy-buttersäureamid
N-(2-Diethylamino-ethyl)-2-nitroxy-propionsäureamid
N-(2-Diethylamino-ethyl)-2-methyl-2-nitroxy-propionsäureamid
N-(2-Diethylamino-ethyl)-2,2-dimethyl-3-nitroxy-propionsäureamid
N-(2-Diethylamino-ethyl)-3,3-dimethyl-5-nitroxy-valeriansäureamid
2-Methyl-2-nitroxy-N-[2-(1-pyrrolidino)-ethyl]-propionsäureamid
2,2-Dimethyl-3-nitroxy-N-[2-(1-pyrrolidino)-ethyl]-propionsäureamid
3-Nitroxy-N-[2-(1-pyrrolidino)-ethyl]-buttersäureamid
4-Nitroxy-N-[2-(1-pyrrolidino)-ethyl]-buttersäureamid
5-Nitroxy-N-[2-(1-pyrrolidino)-ethyl]-valeriansäureamid
2-Nitroxy-N-[2-(1-piperidino)-ethyl]-propionsäureamid
2,2-Dimethyl-3-nitroxy-N-[2-(1-piperidino)-ethyl]-propionsäureamid
4-Nitroxy-N-[2-(1-piperidino)-ethyl]-buttersäureamid
5-Nitroxy-N-[2-(1-piperidino)-ethyl]-valeriansäureamid
3,3-Dimethyl-5-nitroxy-N-[2-(1-piperidino)-ethyl]-valeriansäureamid
N-[2-(4-Morpholino)-ethyl]-2-nitroxy-propionsäureamid
2-Methyl-N-[2-(4-morpholino)-ethyl]-2-nitroxy-propionsäureamid
2,2-Dimethyl-N-[2-(4-morpholino)-ethyl]-3-nitroxy-propionsäureamid
N-(3-Amino-propyl)-2,2-dimethyl-3-nitroxy-propionsäureamid
4-Nitroxy-buttersäurepiperazid
4-Nitroxy-valeriansäurepiperazid
2-Nitroxy-propionsäure-(4-methyl)-piperazid
2-Methyl-2-nitroxy-propionsäure-(4-methyl)-piperazid
2,2-Dimethyl-3-nitroxy-propionsäure-(4-methyl)-piperazid
4-Nitroxy-buttersäure-(4-methyl)-piperazid
5-Nitroxy-valeriansäure-(4-methyl)-piperazid
cis-2-Amino-N-(4-nitroxy-cyclohexyl)-essigsäureamid
cis-2-Amino-N-(4-nitroxy-cyclohexylmethyl)-essigsäureamid
trans-2-Amino-N-(4-nitroxymethyl-cyclohexyl)-essigsäureamid
cis-2-Amino-N-(4-nitroxymethyl-cyclohexyl)-essigsäureamid
trans-2-Dimethylamino-N-(2-nitroxy-cyclohexyl)-essigsäureamid
cis-2-Dimethylamino-N-(4-nitroxy-cyclohexyl)-essigsäureamid
trans-2-Dimethylamino-N-(4-nitroxy-cyclohexylmethyl)-essigsäureamid
trans-2-Dimethylamino-N-(4-nitroxymethyl-cyclohexyl)-essigsäureamid
cis-2-Dimethylamino-N-(4-nitroxymethyl-cyclohexyl)-essigsäureamid
L-trans-2-Amino-N-(4-nitroxy-cyclohexyl)-propionsäureamid
L-cis-2-Amino-N-(4-nitroxy-cyclohexyl)-propionsäureamid
L-trans-2-Amino-N-(4-nitroxy-cyclohexylmethyl)-propionsäureamid
L-cis-2-Amino-N-(4-nitroxy-cyclohexylmethyl)-propionsäureamid
L-trans-2-Amino-N-(4-nitroxymethyl-cyclohexyl)-propionsäureamid
L-cis-2-Amino-N-(4-nitroxymethyl-cyclohexyl)-propionsäureamid
trans-3-Amino-N-(2-nitroxy-cyclohexyl)-propionsäureamid
cis-3-Amino-N-(4-nitroxy-cyclohexyl)-propionsäureamid
trans-3-Amino-N-(4-nitroxy-cyclohexylmethyl)-propionsäureamid
cis-3-Amino-N-(4-nitroxy-cyclohexylmethyl)-propionsäureamid
trans-3-Amino-N-(4-nitroxymethyl-cyclohexyl)-propionsäureamid
cis-3-Amino-N-(4-nitroxymethyl-cyclohexyl)-propionsäureamid
trans-3-Dimethylamino-N-(2-nitroxy-cyclohexyl)-propionsäureamid
cis-3-Dimethylamino-N-(4-nitroxy-cyclohexyl)-propionsäureamid
trans-3-Dimethylamino-N-(4-nitroxy-cyclohexylmethyl)-propionsäureamid
cis-3-Dimethylamino-N-(4-nitroxy-cyclohexylmethyl)-propionsäureamid
trans-3-Dimethylamino-N-(4-nitroxymethyl-cyclohexyl)-propionsäureamid
cis-3-Dimethylamino-N-(4-nitroxymethyl-cyclohexyl)-propionsäureamid
trans-3-Amino-3-methyl-N-(2-nitroxy-cyclohexyl)-buttersäureamid
trans-3-Amino-3-methyl-N-(4-nitroxy-cyclohexyl)-buttersäureamid
cis-3-Amino-3-methyl-N-(4-nitroxy-cyclohexyl)-buttersäureamid
trans-3-Amino-3-methyl-N-(4-nitroxy-cyclohexylmethyl)-buttersäureamid
trans-3-Amino-3-methyl-N-(4-nitroxymethyl-cyclohexyl)-buttersäureamid
cis-3-Amino-3-methyl-N-(4-nitroxymethyl-cyclohexyl)-buttersäureamid
trans-N-(2-Amino-ethyl)-2-(4-nitroxy-cyclohexyl)-essigsäureamid
cis-N-(2-Amino-ethyl)-2-(4-nitroxy-cyclohexyl)-essigsäureamid
trans-N-(2-Amino-2-methyl-propyl)-2-(4-nitroxy-cyclohexyl)-essigsäureamid
cis-N-(2-Amino-2-methyl-propyl)-2-(4-nitroxy-cyclohexyl)-essigsäureamid
trans-N-(2-Dimethylamino-ethyl)-4-nitroxy-cyclohexancarbonsäureamid
trans-N-(2-Dimethylamino-ethyl)-2-(4-nitroxy-cyclohexyl)-essigsäureamid
cis-N-(2-Dimethylamino-ethyl)-2-(4-nitroxy-cyclohexyl)-essigsäureamid
trans-N-(2-Diethylamino-ethyl)-2-(2-nitroxy-cyclohexyl)-essigsäureamid
trans-N-(2-Diethylamino-ethyl)-2-(4-nitroxy-cyclohexyl)-essigsäureamid
cis-N-(2-Diethylamino-ethyl)-2-(4-nitroxy-cyclohexyl)-essigsäureamid
trans-2-(4-Nitroxy-cyclohexyl)-N-[2-(1-pyrrolidino)-ethyl]-essigsäureamid
cis-2-(4-Nitroxy-cyclohexyl)-N-[2-(1-pyrrolidino)-ethyl]-essigsäureamid
trans-2-(4-Nitroxy-cyclohexyl)-N-[2-(1-piperidino)-ethyl]-essigsäureamid
cis-2-(4-Nitroxy-cyclohexyl)-N-[2-(1-piperidino)-ethyl]-essigsäureamid
cis-N-[2-(4-Morpholino)-ethyl)-2-(4-nitroxy-cyclohexyl)-essigsäureamid
trans-N-(3-Amino-propyl)-2-(4-nitroxy-cyclohexyl)-essigsäureamid
cis-N-(3-Amino-propyl)-2-(4-nitroxy-cyclohexyl)-essigsäureamid
trans-N-(3-Amino-2,2-dimethyl-propyl)-2-(4-nitroxy-cyclohexyl)-essigsäureamid
cis-N-(3-Amino-2,2-dimethyl-propyl)-2-(4-nitroxy-cyclohexyl)-essigsäureamid
cis-4-Nitroxy-cyclohexancarbonsäure-piperazid
cis-2-(4-Nitroxy-cyclohexyl)-essigsäurepiperazid
cis-4-Nitroxy-cyclohexancarbonsäure-(4-methyl)-piperazid
cis-2-(4-Nitroxy-cyclohexyl)-essigsäure-(4-methyl)-piperazid
4-Nitroxy-buttersäure-(4-aminocarbonylamino)-piperidid
2,2-Dimethyl-3-nitroxy-propionsäure-(4-aminocarbonylamino)-piperidid
5-Nitroxy-valeriansäure-(4-aminocarbonylamino)-piperidid
4-Nitroxy-buttersäure-(4-aminocarbonyl)-piperazid
trans-N-(2-Nitroxy-cyclohexylmethyl)-harnstoff

### Beispiel 1

### 3-Amino-N-(3-nitroxy-propyl) propionsäureamid fumarat

28.7 g 3-Amino-N-(3-hydroxy-propyl)-propionsäureamid hemioxalat werden unter Rühren bei 5° C in 63 ml 100proz. Salpetersäure eingetragen. Man läßt die Reaktionslösung noch etwa 1 h im Eisbad bei +5° C rühren, gibt dann 1 L Methylenchlorid zu und neutralisiert unter weiterem Rühren mit 234 g Kaliumcarbonat x 1.5 H₂O. Nach einstündigem Rühren im Eisbad werden 100 ml Ethanol zugesetzt und noch etwa 18 h bei Raumtemperatur gerührt. Die Suspension wird abgesaugt und das Filtrat im Vakuum bei max. 20° C abdestilliert. Es verbleiben etwa 32 g Rohbase. Durch Lösen in Ethanol und Zugabe von 17.4 g Fumarsäure erhält man 23 g Fumarat der Titelverbindung vom Schmp. 119-120° C, d.s. 50% d.Th.

Nach dem in Beispiel 1 beschriebenen Verfahren werden in analoger Weise die folgenden Verbindungen hergestellt:
1) 2-Amino-N-(2-nitroxy-ethyl)-essigsäureamid fumarat aus 2-Amino-N-(2-hydroxy-ethyl)-essigsäureamid oxalat
   Ausbeute: 20 %; Lösungsmittel: Ethanol/Diethylether; Schmp. 123-125°C.
2) 2-Amino-N-(1-nitroxy-2-methyl-prop-2-yl)-essigsäureamid fumarat aus 2-Amino-N-(1-hydroxy-2-methyl-prop-2-yl)-essigsäureamid oxalat
   Ausbeute: 70 %; Lösungsmittel: Ethanol; Schmp. 142-145°C.
3) 2-Amino-N-(3-nitroxy-propyl)-essigsäureamid fumarat aus 2-Amino-N-(3-hydroxy-propyl)-essigsäureamid oxalat
   Ausbeute: 38 %; Lösungsmittel: Ethanol; Schmp. 122-124°C
4) 2-Amino-N-(2,2-dimethyl-3-nitroxy-propyl)-essigsäureamid fumarat aus 2-Amino-N-(2,2-dimethyl-3-hydroxy-propyl)-essigsäureamid oxalat
   Ausbeute: 30 %; Lösungsmittel: Ethanol; Schmp. 146-151°C
5) 2-Dimethylamino-N-(2-nitroxy-ethyl)essigsäureamid fumarat aus 2-Dimethylamino-N-(2-hydroxy-ethyl)essigsäureamid oxalat
   Ausbeute: 90 %; Lösungsmittel: Ethanol; Schmp. 111-113°C
6) 2-Amino-N-(2-nitroxy-ethyl)-propionsäureamid fumarat aus 2-Amino-N-(2-hydroxy-ethyl)-propionsäureamid oxalat
   Ausbeute: 15 %; Lösungsmittel: Ethanol; Schmp. 114-120°C
7) 2-Amino-2-methyl-N-(2-nitroxy-ethyl)-propionsäureamid fumarat aus 2-Amino-2-methyl-N-(2-hydroxy-ethyl)-propionsäureamid oxalat
   Ausbeute: 66 %; Lösungsmittel: Ethanol; Schmp. 126°C
8) 2-Amino-2-methyl-N-(3-nitroxy-2-methyl-prop-2-yl)-propionsäure-amid fumarat aus 2-Amino-2-methyl-N-(3-hydroxy-2-methyl-prop-2-yl)-propionsäure-amid oxalat
   Ausbeute: 50 %; Lösungsmittel: Ethanol; Schmp. 137-138°C
9) 2-Amino-2-methyl-N-(3-nitroxy-propyl)-propionsäureamid fumarat aus 2-Amino-2-methyl-N-(3-hydroxy-propyl)-propionsäureamid
   Ausbeute: 22 %; Lösungsmittel: Ethanol; Schmp. 133-134°C
10) 2-Amino-2-methyl-N-(1-nitroxy-but-3-yl)-propionsäureamid fumarat aus 2-Amino-2-methyl-N-(1-hydroxy-but-3-yl)-propionsäureamid oxalat
   Ausbeute: 33 %; Lösungsmittel: Ethanol; Schmp. 142-146°C
11) 3-Amino-N-(2-nitroxy-ethyl)-propionsäureamid fumarat aus 3-Amino-N-(2-hydroxy-ethyl)-propionsäureamid oxalat
   Ausbeute: 22 %; Lösungsmittel: Ethanol; Schmp. 117-119°C
12) 3-Dimethylamino-N-(3-nitroxy-propyl)-propionsäureamid aus 3-Dimethylamino-N-(3-hydroxy-propyl)-propionsäureamid
   Ausbeute: 30 %; Lösungsmittel: Ethanol; Schmp. 82-85°C
13) 3-Amino-3-methyl-N-(2-nitroxy-ethyl)-buttersäureamid oxalat aus 3-Amino-3-methyl-N-(2-hydroxy-ethyl)-buttersäureamid oxalat
   Ausbeute: 30 %; Lösungsmittel: Aceton; Schmp. 117-123°C
14) 4-Amino-N-(2-nitroxy-ethyl)-butter-säureamid fumarat aus 4-Amino-N-(2-hydroxy-ethyl)-butter-säureamid oxalat
   Ausbeute: 25 %; Lösungsmittel: Ethanol; Schmp. 99-100°C
15) 4-Amino-N-(3-nitroxy-propyl)-buttersäureamid fumarat aus 4-Amino-N-(3-hydroxy-propyl)-buttersäureamid oxalat
   Ausbeute: 30 %; Lösungsmittel: Ethanol; Schmp. 112-113°C
16) 4-Amino-4-methyl-N-(2-nitroxy-ethyl)-valeriansäureamid hemifumarat aus 4-Amino-4-methyl-N-(2-hydroxy-ethyl)-valeriansäureamid oxalat
   Ausbeute: 29 %; Lösungsmittel: Aceton; Schmp. 116-119°C
17) 4-Amino-4-methyl-N-(3-nitroxy-propyl)-valeriansäureamid fumarat aus 4-Amino-4-methyl-N-(3-hydroxy-propyl)-valeriansäureamid hemioxalat
   Ausbeute: 68 %; Lösungsmittel: Ethylacetat/Aceton; Schmp. 102°C (Zers.)
18) N-(2-Amino-ethyl)-2-methyl-2-nitroxy-propionsäureamid fumarat aus N-(2-Amino-ethyl)-2-methyl-2-hydroxy-propionsäureamid
   Ausbeute: 26 %; Lösungsmittel: Ethanol; Schmp. 125-127°C
19) N-(2-Amino-ethyl)-2,2-dimethyl-3-nitroxy-propionsäureamid fumarat aus N-(2-Amino-ethyl)-2,2-dimethyl-3-hydroxy-propionsäureamid oxalat
   Ausbeute: 80 %; Lösungsmittel: Ethanol; Schmp. 158-160°C
20) N-(2-Amino-ethyl)-3-nitroxy-buttersäureamid fumarat aus N-(2-Amino-ethyl)-3-hydroxy-buttersäureamid
   Ausbeute: 50 %; Lösungsmittel: Ethanol; Schmp. 126-128°C
21) N-(2-Amino-ethyl)-4-nitroxy-buttersäureamid fumarat aus N-(2-Amino-ethyl)-4-hydroxy-buttersäureamid
   Ausbeute: 63 %; Lösungsmittel: Ethanol; Schmp. 105-106°C
22) N-(2-Amino-ethyl)-2-methyl-4-nitroxy-buttersäureamid fumarat aus N-(2-Amino-ethyl)-2-methyl-4-hydroxy-buttersäureamid oxalat
   Ausbeute: 65 %; Lösungsmittel: Ethylacetat; Schmp. 64-67°C
23) N-(2-Amino-ethyl)-5-nitroxy-valeriansäureamid fumarat aus N-(2-Amino-ethyl)-5-hydroxy-valeriansäureamid oxalat
   Ausbeute: 60 %; Lösungsmittel: Ethanol; Schmp. 75°C
24) N-(2-Amino-2-methyl-propyl)-2-methyl-2-nitroxy-propionsäureamid fumarat aus N-(2-Amino-2-methyl-propyl)-2-methyl-2-hydroxy-propionsäureamid
   Ausbeute: 41 %; Lösungsmittel: Aceton; Schmp. 150-152°C
25) N-(2-Amino-2-methyl-propyl)-2,2-dimethyl-3-nitroxy-propionsäureamid fumarat aus N-(2-Amino-2-methyl-propyl)-2,2-dimethyl-3-hydroxy-propionsäureamid oxalat
   Ausbeute: 52 %; Lösungsmittel: Ethylacetat; Schmp. 150-155°C
26) N-(2-Amino-2-methyl-propyl)-3-nitroxy-buttersäureamid fumarat aus N-(2-Amino-2-methyl-propyl)-3-hydroxy-buttersäureamid oxalat
   Ausbeute: 53 %; Lösungsmittel: Ethylacetat; Schmp. 128-130°C
27) N-(2-Amino-2-methyl-propyl)-4-nitroxy-buttersäureamid fumarat aus N-(2-Amino-2-methyl-propyl)-2-hydroxy-buttersäureamid oxalat
   Ausbeute: 38 %; Lösungsmittel: Ethylacetat; Schmp. 175-178°C
28) N-(2-Amino-2-methyl-propyl)-5-nitroxy-capronsäureamid fumarat aus N-(2-Amino-2-methyl-propyl)-5-hydroxy-capronsäureamid oxalat
   Ausbeute: 85 %; Lösungsmittel: Ethylacetat; Schmp. 60-65°C
29) N-(2-Amino-2-methyl-propyl)-5-nitroxy-valeriansäureamid hemifumarat aus N-(2-Amino-2-methyl-propyl)-5-hydroxy-valeriansäureamid oxalat
   Ausbeute: 17 %; Lösungsmittel: Ethylacetat/2-Propanol; Schmp. 128-132°C
30) N-(2-Amino-2-methyl-propyl)-3,3-dimethyl-5-nitroxy-valeriansäureamid fumarat aus N-(2-Amino-2-methyl-propyl)-3,3-dimethyl-5-hydroxy-valeriansäureamid oxalat
   Ausbeute: 38 %; Lösungsmittel: Ethylacetat; Schmp. 104-107°C
31) N-(2-Amino-2-methyl-propyl)-6-nitroxy-capronsäureamid fumarat aus N-(2-Amino-2-methyl-propyl)-6-hydroxy-capronsäureamid oxalat
   Ausbeute: 21 %; Lösungsmittel: Ethylacetat/Aceton; Schmp. 95-100°C
32) N-(2-Dimethylamino-ethyl)-5-nitroxy-valeriansäureamid cyclaminat aus N-(2-Dimethylamino-ethyl)-5-hydroxy-valeriansäureamid oxalat
   Ausbeute: 18 %; Lösungsmittel: Ethylacetat; Schmp. 58-60°C
33) N-(2-Diethylamino-ethyl)-4-nitroxy-buttersäureamid fumarat aus N-(2-Diethylamino-ethyl)-4-hydroxy-buttersäureamid oxalat
   Ausbeute: 24 %; Lösungsmittel: Ethylacetat; Schmp. 148-150°C
34) 2-Nitroxy-N-[2-(1-pyrrolidino)-ethyl]-propionsäureamid fumarat aus 2-Hydroxy-N-[2-(1-pyrrolidino)-ethyl]-propionsäureamid oxalat
   Ausbeute: 60 %; Lösungsmittel: Ethanol; Schmp. 98-100°C
35) N-(3-Amino-propyl)-2-nitroxy-propionsäureamid fumarat aus N-(3-Amino-propyl)-2-hydroxy-propionsäureamid oxalat
   Ausbeute: 24 %; Lösungsmittel: Ethanol; Schmp. 118-121°C
36) N-(3-Amino-propyl)-2-methyl-2-nitroxy-propionsäureamid fumarat aus N-(3-Amino-propyl)-2-methyl-2-hydroxy-propionsäureamid oxalat
   Ausbeute: 15 %; Lösungsmittel: Ethanol; Schmp. 147-149°C
37) N-(3-Amino-propyl)-4-nitroxy-buttersäureamid fumarat aus N-(3-Amino-propyl)-4-hydroxy-buttersäureamid oxalat
   Ausbeute: 60 %; Lösungsmittel: Ethanol; Schmp. 102-105°C
38) N-(3-Amino-2,2-dimethyl-propyl)-2-methyl-2-nitroxy-propionsäureamid hemifumarat aus N-(3-Amino-2,2-dimethyl-propyl)-2-methyl-2-hydroxy-propionsäureamid oxalat
   Ausbeute: 57 %; Lösungsmittel: Ethanol/Diethylether; Schmp. 156-158°C
39) N-(3-Amino-2,2-dimethyl-propyl)-2,2-dimethyl-3-nitroxy-propionsäureamid hemifumarat aus N-(3-Amino-2,2-dimethyl-propyl)-2,2-dimethyl-3-hydroxy-propionsäureamid oxalat
   Ausbeute: 30 %; Lösungsmittel: Ethanol/Diethylether; Schmp. 154-157°C
40) N-(3-Amino-2,2-dimethyl-propyl)-5-nitroxy-valeriansäureamid fumarat aus N-(3-Amino-2,2-dimethyl-propyl)-5-hydroxy-valeriansäureamid oxalat
   Ausbeute: 70 %; Lösungsmittel: Ethylacetat; Schmp. 56-60°C
41) trans-2-Amino-N-(2-nitroxy-cyclohexyl)-essigsäureamid fumarat aus trans-2-Amino-N-(2-hydroxy-cyclohexyl)-essigsäureamid
   Ausbeute: 16 %; Lösungsmittel: Aceton; Schmp. 166-168°C
42) trans-2-Amino-N-(4-nitroxy-cyclohexyl)-essigsäureamid fumarat aus trans-2-Amino-N-(4-hydroxy-cyclohexyl)-essigsäureamid
   Ausbeute: 20 %; Lösungsmittel: Ethanol; Schmp. 160-162°C
43) trans-2-Amino-N-(2-nitroxy-cyclohexylmethyl)-essigsäureamid fumarat aus trans-2-Amino-N-(2-hydroxy-cyclohexylmethyl)-essigsäureamid
   Ausbeute: 10 %; Lösungsmittel: Ethanol; Schmp. 125-127°C
44) trans-2-Amino-N-[2-(2-nitroxy-cyclohexyl)-ethyl]-essigsäureamid fumarat aus trans-2-Amino-N-[2-(2-hydroxy-cyclohexyl)-ethyl]-essigsäureamid
   Ausbeute: 19 %; Lösungsmittel: Ethanol; Schmp. 86-90°C
45) trans-2-Amino-2-methyl-N-(2-nitroxy-cyclohexylmethyl)-propionsäureamid fumarat aus trans-2-Amino-2-methyl-N-(2-hydroxy-cyclohexylmethyl)-propionsäureamid oxalat
   Ausbeute: 20 %; Lösungsmittel: Ethanol; Schmp. 174-177°C
46) trans-2-Amino-2-methyl-N-[2-(2-nitroxy-cyclohexyl]-ethyl]-propionsäureamid fumarat aus trans-2-Amino-2-methyl-N-[2-(2-hydroxy-cyclohexyl)-ethyl]-propionsäureamid oxalat
   Ausbeute: 36 %; Lösungsmittel: Ethanol; Schmp. 167-170°C
47) trans-3-Amino-N-(4-nitroxy-cyclohexyl)-propionsäureamid fumarat aus trans-3-Amino-N-(4-hydroxy-cyclohexyl)-propionsäureamid
   Ausbeute: 27 %; Lösungsmittel: Ethylacetat; Schmp. 105-110°C
48) trans-N-(2-Amino-ethyl)-4-nitroxy-cyclohexancarbonsäureamid fumarat aus trans-N-(2-Amino-ethyl)-4-hydroxy-cyclohexancarbonsäureamid
   Ausbeute: 21 %; Lösungsmittel: Ethanol; Schmp. 84-86°C
49) trans-N-(2-Amino-ethyl)-2-(2-nitroxy-cyclohexyl)-essigsäureamid fumarat aus trans-N-(2-Amino-ethyl)-2-(2-hydroxy-cyclohexyl)-essigsäureamid
   Ausbeute: 85 %; Lösungsmittel: Ethanol; Schmp. 78-80°C
50) trans-N-(2-Amino-2-methyl-propyl)-4-nitroxy-cyclohexancarbonsäureamid fumarat aus trans-N-(2-Amino-2-methyl-propyl)-2-hydroxy-cyclohexancarbonsäureamid
   Ausbeute: 61 %; Lösungsmittel: Ethylacetat; Schmp. 140-143°C
51) cis-N-(2-Amino-2-methyl-propyl)-4-nitroxy-cyclohexancarbonsäureamid hemifumarat aus trans-N-(2-Amino-2-methyl-propyl)-2-hydroxy-cyclohexancarbonsäureamid
   Ausbeute: 45 %; Lösungsmittel: Ethanol; Schmp. 182-184°C
52) trans-N-(2-Amino-2-methyl-propyl)-2-(2-nitroxy-cyclohexyl)-essigsäureamid fumarat aus trans-N-(2-Amino-2-methyl-propyl)-2-(2-hydroxy-cyclohexyl)-essigsäureamid
   Ausbeute: 38 %; Lösungsmittel: Aceton; Schmp. 150-152°C
53) trans-N-(2-Dimethylamino-ethyl)-2-(2-nitroxy-cyclohexyl)-essigsäureamid cyclaminat aus trans-N-(2-Dimethylamino-ethyl)-2-(2-hydroxy-cyclohexyl)essigsäureamid oxalat
   Ausbeute: 54 %; Lösungsmittel: Ethylacetat; Schmp. 90-93°C
54) trans-N-(2-Diethylamino-ethyl)-4-nitroxy-cyclohexancarbonsäureamid fumarat aus trans-N-(2-Diethylamino-ethyl)-4-hydroxy-cyclohexancarbonsäureamid oxalat
   Ausbeute: 20 %; Lösungsmittel: Aceton; Schmp. 93-96°C
55) trans-N-[2-(1-Pyrrolidinyl)-ethyl]-4-nitroxy-cyclohexancarbonsäureamid fumarat aus trans-N-[2-(1-Pyrrolidinyl)-ethyl]-4-hydroxy-cyclohexancarbonsäureamid
   Ausbeute: 17 %; Lösungsmittel: Ethanol; Schmp. 126-127°C
56) cis-N-[2-(1-Pyrrolidinyl)-ethyl]-4-nitroxy-cyclohexancarbonsäureamid maleinat aus cis-N-[2-(1-Pyrrolidinyl)-ethyl]-4-hydroxy-cyclohexancarbonsäureamid
   Ausbeute: 33 %; Lösungsmittel: Aceton/Diethylether; Schmp. 91-93°C
57) trans-N-[2-(1-Piperidinyl)-ethyl]-4-nitroxy-cyclohexancarbonsäureamid fumarat aus trans-N-[2-(1-Piperidinyl)-ethyl]-4-hydroxy-cyclohexancarbonsäureamid
   Ausbeute: 15 %; Lösungsmittel: Ethanol; Schmp. 154-155°C
58) cis-N-[2-(1-Piperidinyl)-ethyl]-4-nitroxy-cyclohexancarbonsäureamid maleinat aus cis-N-[2-(1-Piperidinyl)-ethyl]-4-hydroxy-cyclohexancarbonsäureamid
   Ausbeute: 15 %; Lösungsmittel: Aceton/Diethylether; Schmp. 98-100°C
59) cis-N-(3-Amino-propyl)-4-nitroxy-cyclohexancarbonsäureamid hemifumarat aus cis-N-(3-Amino-propyl)-4-hydroxy-cyclohexancarbonsäureamid
   Ausbeute: 10 %; Lösungsmittel: Ethanol; Schmp. 148-151°C
60) trans-2-Dimethylamino-N-(4-nitroxy-cyclohexyl)-essigsäureamid fumarat aus trans-2-Dimethylamino-N-(4-hydroxy-cyclohexyl)-essigsäureamid oxalat
   Ausbeute: 62 %; Lösungsmittel: Ethanol; Schmp. 146-147°C
61) trans-N-[2-(4-Morpholino)-ethyl)-4-nitroxy]-cyclohexancarbonsäureamid hemifumarat aus trans-N-[2-(4-Morpholino)-ethyl) 4-hydroxy]-cyclohexancarbonsäureamid oxalat
   Ausbeute: 35 %; Lösungsmittel: Ethanol; Schmp. 146-147°C
62) trans-4-Nitroxy-cyclohexancarbonsäure-(4-methyl)-piperazid fumarat aus trans-4-Hydroxy-cyclohexancarbonsäure-(4-methyl)-piperazid oxalat
   Ausbeute: 60 %; Lösungsmittel: Ethanol; Schmp. 168-170°C
63) trans-N-(3-Amino-2,2-dimethyl-propyl)-4-nitroxy-cyclohexancarbonsäureamid fumarat aus trans-N-(3-Amino-2,2-dimethyl-propyl)-4-hydroxy-cyclohexancarbonsäureamid oxalat
   Ausbeute: 25 %; Lösungsmittel: Ethanol; Schmp. 126-127°C
64) 2-Dimethylamino-N-(3-nitroxypropyl)-essigsäureamid fumarat aus 2-Dimethylamino-N-(3-hydroxypropyl)-essigsäureamid
   Ausbeute: 51 %; Lösungsmittel: Ethanol; Schmp. 112-115°C
65) 3-Dimethylamino-N-(2-nitroxyethyl)-propionsäureamid fumarat aus 3-Dimethylamino-N-(2-hydroxyethyl)-propionsäureamid
   Ausbeute: 30 %; Lösungsmittel: Ethanol; Schmp. 105-107°C
66) trans-3-Dimethylamino-N-(4-nitroxy-cyclohexyl)-propionsäureamid fumarat aus trans-3-Dimethylamino-N-(4-hydroxy-cyclohexyl)-propionsäureamid
   Ausbeute: 30 %; Lösungsmittel: Ethanol; Schmp. 154-156°C
67) N-[2-(4-Morpholino)-ethyl]-4-nitroxy-buttersäureamid fumarat aus N-[2-(4-Morpholino)-ethyl]-4-hydroxy-buttersäureamid
   Ausbeute: 50 %; Lösungsmittel: Ethanol; Schmp. 96-97°C
68) cis-N-(2-Dimethylamino-ethyl)-4-nitroxy-cyclohexancarbonsäureamid fumarat aus cis-N-(2-Dimethylamino-ethyl)-4-hydroxy-cyclohexancarbonsäureamid
   Ausbeute: 25 %; Lösungsmittel: Ethylacetat; Schmp. 78-80°C
69) cis-N-(2-Diethylamino-ethyl)-4-nitroxy-cyclohexancarbonsäureamid aus cis-N-(2-Diethylamino-ethyl)-4-hydroxy-cyclohexancarbonsäureamid
   Ausbeute: 20 %; Lösungsmittel: Diethylether; Schmp. 50-51°C
70) cis-N-[2-(4-Morpholino)-ethyl]-4-nitroxy-cyclohexancarbonsäureamid fumarat aus cis-N-[2-(4-Morpholino)-ethyl]-4-hydroxy-cyclohexancarbonsäureamid
   Ausbeute: 10 %; Lösungsmittel: Ethanol; Schmp. 114-116°C
71) cis-N-(3-Amino-2,2-dimethyl-propyl)-4-nitroxycyclohexancarbonsäureamid fumarat aus cis-N-(3-Amino-2,2-dimethyl-propyl)-4-hydroxycyclohexan-carbonsäureamid
   Ausbeute: 50 %; Lösungsmittel: Ethanol/Ethylacetat; Schmp. 130-135°C
72) trans-4-Nitroxy-cyclohexan-carbonsäure-piperazid hemifumarat aus trans-4-Hydroxy-cyclohexan-carbonsäure-piperazid
   Ausbeute: 75 %; Lösungsmittel: Ethanol; Schmp. 136-138°C

### Beispiel 2

### N-(2-Amino-ethyl)-4-nitroxy-buttersäureamid oxalat

In einem 1-l-Dreihalskolben werden 21.9 g N-(2-Amino-ethyl)-4-hydroxy-buttersäureamid in 300 ml Acetonitril suspendiert und im Eisbad auf +5°C abgekühlt. Durch Zugabe von 9.5 ml 100 proz. Salpetersäure in 100 ml Acetonitril (im Eisbad bei 5-10° C gemischt) wird die Base in das salpetersaure Salz überführt. Zu dieser Suspension wird nun eine im Eisbad gemischte Lösung von Acetylnitrat in Acetonitril innerhalb 30 Minuten bei 5-7° C zugetropft, wobei eine klare Lösung entsteht. Man läßt noch 1 h im Eisbad rühren.

1 l tert.-Butylmethylether wird in einem 2-l-Dreihalskolben im Eisbad auf 5-7° C abgekühlt. Dazu läßt man unter Rühren die oben erhaltene Reaktionslösung in dünnem Strom zufließen. Das Produkt fällt als salpetersaures Salz ölig aus, die überstehende Lösung wird dekantiert. Zum öligen Rückstand gibt man eine Mischung aus 675 ml Methylenchlorid und 75 ml Ethanol und neutralisiert mit 31.1 g Kaliumcarbonat, dem zuvor 4.1 ml Wasser zugesetzt wurden. Man läßt über Nacht bei Raumtemperatur rühren.
Man saugt von den anorganischen Salzen ab und wäscht 3mal mit je 50 ml Methylenchlorid nach. Zum Filtrat gibt man eine Lösung von 13.5 g Oxalsäure in 40 ml Ethanol. Der Niederschlag wird abgesaugt, mit Methylenchlorid und Ether gewaschen und getrocknet. Man erhält 25.8 g Kristalle vom Schmp. 102-103° C, d.s. 61% d.Th.

In analoger Weise wurden erhalten:
1) 2-Amino-2-methyl-N-(2-nitroxy-propyl)-propionsäureamid hemifumarat aus 2-Amino-2-methyl-N-(2-hydroxy-propyl)-propionsäureamid oxalat
   Ausbeute: 16 %; Lösungsmittel: Ethanol; Schmp. 128-131°C
2) 2-Amino-2-methyl-N-(2,2-dimethyl-3-nitroxy-propyl)-propionsäureamid hemifumarat aus 2-Amino-2-methyl-N-(2,2-dimethyl-3-hydroxy-propyl)-propionsäureamid oxalat
   Ausbeute: 27 %; Lösungsmittel: Ethanol; Schmp. 157-159°C
3) N-(3-Amino-2,2-dimethyl-propyl)-4-nitroxy-buttersäureamid fumarat aus N-(3-Amino-2,2-dimethyl-propyl)-4-hydroxy-buttersäureamid oxalat
   Ausbeute: 15 %; Lösungsmittel: Ethanol; Schmp. 91-93°C
4) trans-2-Amino-2-methyl-N-(2-nitroxy-cyclohexyl)-propionsäureamid hemifumarat aus trans-2-Amino-2-methyl-N-(2-hydroxy-cyclohexyl)-propionsäureamid oxalat
   Ausbeute: 20 %; Lösungsmittel: Ethanol; Schmp. 159-164°C
5) 2-Amino-N-(3-nitroxy-propyl)-propionsäureamid benzoat aus 2-Amino-N-(3-hydroxy-propyl)-propionsäureamid oxalat
   Ausbeute: 20 %; Lösungsmittel: Ethylacetat; Schmp. 120-125°C
6) trans-2-Amino-2-methyl-N-(4-nitroxy-cyclohexyl)-propionsäureamid cyclaminat aus trans-2-Amino-2-methyl-N-(4-hydroxy-cyclohexyl)-propionsäureamid oxalat
   Ausbeute: 75 %; Lösungsmittel: Ethanol; Schmp. 188-190°C
7) trans-2-Amino-2-methyl-N-(2-nitroxy-cyclohexyl-methyl)-propionsäureamid fumarat aus trans-2-Amino-2-methyl-N-(2-hydroxy-cyclohexyl-methyl)-propionsäureamid
   Ausbeute: 85 %; Lösungsmittel: Ethanol; Schmp. 195-197°C
8) trans-2-Amino-2-methyl-N-(4-nitroxy-methyl-cyclohexyl)-propionsäureamid cyclaminat aus trans-2-Amino-2-methyl-N-(4-hydroxy-methyl-cyclohexyl)-propionsäureamid
   Ausbeute: 45 %; Lösungsmittel: Ethanol; Schmp. 182-184°C
9) 2-Dimethylamino-N-(2-methyl-2-nitroxy-propyl)-essigsäureamid fumarat aus 2-Dimethylamino-N-(2-methyl-2-hydroxy-propyl)-essigsäureamid
   Ausbeute: 54 %; Lösungsmittel: Ethanol; Schmp. 102-103°C
10) N-(3-Amino-2,2-dimethyl-propyl)-2-nitroxy-propionsäureamid fumarat aus N-(3-Amino-2,2-dimethyl-propyl)-2-hydroxy-propionsäureamid
   Ausbeute: 10 %; Lösungsmittel: Ethanol/Diethylether; Schmp. 89-91°C
11) trans-2-Amino-N-(4-nitroxy-cyclohexylmethyl)-essigsäureamid fumarat hydrat aus trans-2-Amino-N-(4-hydroxy-cyclohexyl-methyl)-essigsäureamid
   Ausbeute: 44 %; Lösungsmittel: 2-Propanol; Schmp. 132-135°C
12) cis-N-(2-Amino-ethyl)-4-nitroxy-cyclohexancarbonsäureamid fumarat aus cis-N-(2-Amino-ethyl)-4-hydroxy-cyclohexancarbonsäureamid
   Ausbeute: 47 %; Lösungsmittel: Ethanol; Schmp. 126-128°C
13) trans-N-(3-Amino-propyl)-4-nitroxy-cyclohexancarbonsäureamid fumarat aus trans-N-(3-Amino-propyl)-4-hydroxy-cyclohexancarbonsäureamid
   Ausbeute: 16 %; Lösungsmittel: Ethanol; Schmp. 140-142°C

### Beispiel 3

### 2-Aminocarbonylamino-2-methyl-N-(2-nitroxy-ethyl)-propionsäureamid

0.77 g 2-Amino-2-methyl-N-(2-nitroxy-ethyl)-propionsäureamid fumarat werden in 7.5 ml Methanol und 2.5 ml Wasser gelöst und 0.81 g Kaliumcyanat zugegeben. Man rührt 10 Minuten bei Raumtemperatur und gibt dann 0.30 g Essigsäure zu. Nachdem über Nacht gerührt wurde, fällt man durch Zugabe von 25 ml Ethylacetat die anorganischen Salze aus und saugt ab. Das Filtrat wird über Natriumsulfat getrocknet, abgesaugt und eingeengt. Es verbleiben 0.60 g Rohprodukt. Dieses wird mit Ether verrieben und abgesaugt. Man erhält 0.40 g der Titelverbindung vom Schmelzpunkt 98-100 °C, d.s. 68% d.Th.

In analoger Weise wird erhalten:
1) N-Aminocarbonyl-(3-nitroxy)-propylamin aus 3-Nitroxy-propylamin und Kaliumcyanat
   Ausbeute: 35 %; Lösungsmittel: Ethylacetat; Schmp. 77-79°C
2) 2,2-Dimethyl-3-nitroxy-propionsäure-(4-amino-carbonyl)-piperazid aus 2,2-Dimethyl-3-nitroxy-propion-säure-piperazid
   Ausbeute: 60 %; Lösungsmittel: Ethylacetat/Diethylether; Schmp. 148-150°C
3) 5-Nitroxy-valeriansäure-(4-aminocarbonyl)-piperazid aus 5-Nitroxy-valeriansäure-piperazid
   Ausbeute: 11 %; Lösungsmittel: Ethylacetat; Schmp. 106-107°C
4) trans-4-Nitroxy-cyclohexan-carbonsäure-(4-aminocarbonyl)-piperazid aus trans-4-Nitroxy-cyclohexan-carbonsäure-piperazid
   Ausbeute: 20 %; Lösungsmittel: Ethanol; Schmp. 180-182°C
5) trans-N-(2-Nitroxy-cyclohexyl)-harnstoff aus trans-N-2-Nitroxy-cyclohexylamin
   Ausbeute: 46 %; Lösungsmittel: Ethanol; Schmp. 136-139°C
6) trans-N-(4-Nitroxy-cyclohexyl)-harnstoff aus trans-N-4-Nitroxy-cyclohexylamin
   Ausbeute: 67 %; Lösungsmittel: 2-Propanol; Schmp. 181-183°C
7) trans-N-(4-Nitroxy-cyclohexylmethyl)-harnstoff aus trans-N-4-Nitroxy-cyclohexylmethylamin
   Ausbeute: 40 %; Lösungsmittel: Ethylacetat/Diethylether; Schmp. 82-84°C
8) N-Aminocarbonyl-(2,2-dimethyl)-3-nitroxy-propylamin aus 2,2-Dimethyl-3-nitroxy-propylamin
   Ausbeute: 74 %; Lösungsmittel: Diethylether; Schmp. 73-75°C

### Beispiel 4

### 2-Isopropylaminocarbonylamino-2-methyl-N-(2-nitroxy-ethyl)-propionsäureamid

1.9 g 2-Amino-2-methyl-N-(2-nitroxy-ethyl)-propionsäureamid werden in 10 ml Ethylacetat gelöst und unter Kühlen mit Eiswasser bei 5-10 °C mit 1.1 ml Isopropylisocyanat versetzt. Man rührt 2 h bei 10 °C, saugt ab und wäscht mit Ether. Es verbleiben 1.4 g der Titelverbindung vom Schmelzpunkt 120-122 °C, d.s. 50% d.Th.

In analoger Weise werden erhalten:
1) N-(2-Isopropylaminocarbonylamino-ethyl)-4-nitroxy-buttersäureamid aus N-(2-Amino-ethyl)-4-nitroxy-buttersäureamid und Isopropylisocyanat
   Ausbeute: 50 %; Lösungsmittel: Ethylacetat/Diethylether; Schmp. 133-135°C

### Beispiel 5

Die Ausgangsverbindungen werden wie folgt hergestellt:

### a) 3-Amino-N-(3-hydroxy-propyl)-propionsäureamid hemioxalat

113 g 2-Cyanessigsäureethylester werden in 1 l Methylenchlorid gelöst und mit 75.1 g 3-Amino-1-propanol versetzt. Nach 3 d rühren bei Raumtemperatur wird im Vacuum abdestilliert. Es verbleiben 153 g Rohprodukt von 2-Cyan-N-(3-hydroxy-propyl)-essigsäureamid, d.s. ca. 100 % d.Th..

61 g dieses Produkts werden in 300 ml Methanol gelöst, mit 300 ml flüssigem Ammoniak versetzt und mit 20 g Raney-Nickel bei 100 b Wasserstoffdruck und Raumtemperatur hydriert. Nach Absaugen des Katalysators wird das Lösungsmittel im Vacuum abdestilliert, der Rückstand in 400 ml Ethanol gelöst und mit 19.4 g Oxalsäure in 200 ml Ethanol versetzt. Nach Stehen über Nacht wird abgesaugt. Es verbleiben 64.2 g Hemioxalat der Titelverbindung vom Schmp. 140-142°C, d.s. 78 % d.Th..

### b) 2-Amino-2-methyl-N-(3-hydroxy-propyl)-propionsäureamid

30 g 3-Amino-1-propanol werden mit 129 g 2-Methyl-2-nitropropionsäuremethylester versetzt und 8 h im Ölbad auf 120 °C erhitzt. Man destilliert im Vacuum bei 15 Torr. den Überschuß an Ester ab. Es verbleiben im Rückstand 76 g Rohprodukt von 2-Methyl-2-nitro-N-(3-hydroxy-propyl)-propionsäureamid.

Dieses wird ohne weitere Reinigung in 1 l Methanol gelöst und bei Raumtemperatur und 1 bar Wasserstoffdruck über Raney-Nickel hydriert. Man saugt vom Katalysator ab und destilliert im Vacuum das Lösungsmittel ab. Es verbleiben etwa 65 g Rohbase. Diese wird in 650 ml Ethanol gelöst und mit 18 g wasserfreier Oxalsäure versetzt. Nach Stehen über Nacht wird abgesaugt. Man erhält 70 g Hemioxalat der Titelverbindung vom Schmp. 156-158 °C, d.s. 85 % d.Th..

### c) Trans-2-Amino-N-(4-hydroxy-cyclohexyl)-essigsäureamid

11.5 g (0.06 mol) trans-2-Chlor-N-(4-hydroxy-cyclohexyl)-essigsäureamid werden in 100 ml Methanol gelöst und mit 200 ml flüssigem Ammoniak 20 h bei 50 °C im Autoklaven geschüttelt. Nach Einengen des Lösungsmittels wird abgesaugt. Es verbleiben 9.7 g der Titelverbindung vom Schmp. 110 - 114 °C, d.s. 94 % d.Th.

Analog werden erhalten:
c1) trans-3-Amino-N-(4-hydroxy-cyclohexyl)-propionsäureamid viskoser Sirup, 60 % Ausbeute.
c2) mit Dimethylamin werden erhalten:
   trans-2-Dimethylamino-D-(4-hydroxy-cyclohexyl)-essigsäureamid oxalat
   Fp 148 - 150 °C (Ethanol), 89 % Ausbeute

### d) trans-2-Chlor-N-(4-hydroxy-cyclohexyl)-essigsäureamid

15.1 g (0.1 mol) trans-4-Aminocyclohexanolhydrochlorid werden in 100 ml Wasser suspendiert und durch Zugabe von 50 ml 2 N Natronlauge auf ein pH von 12.5 eingestellt. Dann tropft man 9.5 ml (0.12 mol) Chloressigsäurechlorid zu und hält durch gleichzeitiges Zutropfen von 2 N Natronlauge den pH bei 12 - 12.5. Nach Ende der Reaktion wird mit n-Butanol extrahiert und die organische Phase im Vacuum abdestilliert. Der Rückstand wird nochmals in Ethanol gelöst, filtriert, eingeengt und mit Ether verrieben. Nach Absaugen verbleiben 12.9 g der Titelverbindung vom Schmp. 149-151 °C, d.s. 67 % d.Th.
d1) Analog werden mit 3-Chlor-propionsäurechlorid erhalten:
   trans-3-Chlor-N-(4-hydroxy-cyclohexyl)-propionsäureamid

73 % Ausbeute Schmp. 153-157 °C (Ethylacetat)

## Patentansprüche

1. Arzneimittel enthaltend mindestens ein Nitroxyalkylamin-Derivat der allgemeinen Formel I 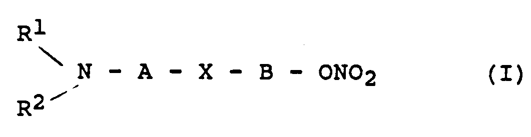 in der
R¹ Wasserstoff, eine geradkettige oder verzweigte, gesättigte oder ungesättigte C₁-C₈-Alkylgruppe, eine C₃-C₈-Cycloalkyl-oder C₈-Cycloalkenylgruppe, eine Aminocarbonyl-, C₁-C₆-Alkylaminocarbonyl- oder Di-C₁-C₆-alkyl-aminocarbonylgruppe bedeutet,
R² Wasserstoff, eine geradkettige oder verzweigte, gesättigte oder ungesättigte C₁-C₆-Alkylgruppe, oder eine C₃-C₈-Cycloalkyl- oder C₈ Cycloalkenylgruppe bedeutet, oder R² zusammen mit R¹ und dem Stickstoffatom, an das sie gebunden sind, einen heteroaliphatischen Ring mit bis zu 6 C-Atomen bilden, worin ein oder zwei C-Atome auch durch Stickstoff-oder Sauerstoffatome ersetzt sein können,
A einen Valenzstrich oder eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
B eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
X eine -NR³-CO-Gruppe oder -CO-NR³-Gruppe bedeutet,
wobei
R³ Wasserstoff oder eine gesättigte oder ungesättigte Alkyl-Gruppe von 1-6 C-Atomen bedeutet, oder für den Fall, daß X die Gruppe -NR³-CO- darstellt, R³ gemeinsam mit den Stickstoffatom und einem C-Atom der Gruppe A einen heterocyclischen Ring mit 4 bis 6 C-Atomen aufspannt, oder R³ gemeinsam mit R², A und den beiden Stickstoffatomen einen heterocyclischen Ring mit 3 bis 5 C-Atomen aufspannt
oder deren optisch aktiven Formen oder physiologisch verträglichen Salze, sowie pharmazeutisch übliche Träger- und Hilfsstoffe.

2. Arzneimittel enthaltend mindestens ein Nitroxyalkylamin-Derivat der Formel I gemäß Anspruch 1, in der
R¹ ein Wasserstoffatom, eine geradkettige oder verzweigte C₁-C₈-Alkylgruppe, eine Aminocarbonyl- oder C₁-C₆-Alkylaminocarbonylgruppe,
R² ein Wasserstoffatom, eine geradkettige oder verzweigte C₁-C₆-Alkylgruppe bedeutet,
oder R¹ und R² zusammen mit dem Stickstoffatom, an das sie gebunden sind, einen Aziridin-, Azetidin-, Pyrrolidin-, Piperidin-, Perhydroazepin-, Piperazin- oder Morpholinring bilden.

3. Arzneimittel enthaltend mindestens ein Nitroxyalkylamin-Derivat der Formel I gemäß Anspruch 1 oder 2, in der
A einen Valenzstrich oder eine Methylmethylen, 1-Methylethylen, Dimethylmethylen, 1,1-Dimethyl-ethylen, 1,1-Dimethyl-trimethylen, 1-Methyl-trimethylen, 1,1-Dimethylmethylen, 1-Ethylmethylen, 1,1-Dimethyl-tetramethylen, 1,2-Dimethyl-trimethylen, 2,2-Dimethyl-trimethylen, 3,3-Dimethyl-trimethylen, 1,1-Di-methyl-pentamethylen, 1,1-Dimethyl-hexamethylen oder 1-Methyl-tetramethylen darstellt.

4. Arzneimittel enthaltend mindestens ein Nitroxyalkylamin-Derivat gemäß einem der Ansprüche 1-3, in der
B Ethylen, 1-Methyl-ethylen, 2-Methyl-ethylen, Trimethylen, 1-Methyl-trimethylen, 3-Methyl-trimethylen, Tetramethylen, Pentamethylen, 1-Methyl-tetra-methylen, 1-Ethyl-trimethylen, Hexamethylen, 1-Methyl-penta-methylen, 1-Ethyl-tetramethylen, 1-n-Propyl-trimethylen, 1,1-Dimethyl-ethylen, Dimethyl-methylen, 1,1-Di-methyl-trimethylen, 2,2-Dimethyl-trimethylen, 2,2-Dimethyl-tetramethylen oder 1,1-Dimethyl-hexamethylen oder eine -CH₂-Gruppe der Kette B durch die Cyclopentylen-Gruppe, die 1,2-, 1,3- oder 1,4-Cyclohexylen-Gruppe, die Methylen-1,2-cyclohexylen-Gruppe, die Methylen-1,3-cyclo-hexylen-Gruppe oder die Methylen-1,4-cyclohexylen-Gruppe ersetzt ist, wobei die Konfiguration der Cycloalkylen-Gruppe jeweils cis oder trans sein kann, bedeutet.

5. Arzneimittel enthaltend mindestens ein Nitroxyalkylamin-Derivat der allgemeinen Formel I gemäß einem der Ansprüche 1-4
in der
R³ ein Wasserstoffatom, eine C₁-C₆-Alkyl-, C₂-C₆-Alkenylgruppe oder R³ gemeinsam mit dem Stickstoffatom und einem C-Atom der Gruppe A einen Pyrrolidin- oder Piperidinring bildet, oder R³ gemeinsam mit R², A und den beiden Stickstoffatomen einen Piperazinring bilden.

6. Nitroxyalkylamin-Derivate der allgemeinen Formel I 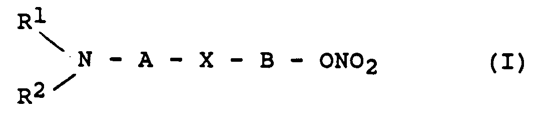 in der
R¹ Wasserstoff, eine geradkettige oder verzweigte, gesättigte oder ungesättigte C₁-C₈-Alkylgruppe, eine C₃-C₈-Cycloalkyl-oder C₈-Cycloalkenylgruppe, eine Aminocarbonyl-, C₁-C₆-Alkylaminocarbonyl- oder Di-C₁-C₆-alkyl-aminocarbonylgruppe bedeutet,
R² Wasserstoff, eine geradkettige oder verzweigte, gesättigte oder ungesättigte C₁-C₆-Alkylgruppe, oder eine C₃-C₈-Cycloalkyl- oder C₈ Cycloalkenylgruppe bedeutet, oder R² zusammen mit R¹ und dem Stickstoffatom, an das sie gebunden sind, einen heteroaliphatischen Ring mit bis zu 6 C-Atomen bilden, worin ein oder zwei C-Atome auch durch Stickstoff-oder Sauerstoffatome ersetzt sein können,
A einen Valenzstrich oder eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
B eine geradkettige oder verzweigte Alkylenkette von 1-8 C-Atomen, wobei eine -CH₂-Gruppe durch eine Cycloalkylen-Gruppe von 3-7 C-Atomen ersetzt sein kann, bedeutet,
X eine -NR³-CO-Gruppe oder -CO-NR³-Gruppe bedeutet,
wobei
R³ Wasserstoff oder eine gesättigte oder ungesättigte Alkyl-Gruppe von 1-6 C-Atomen bedeutet, oder für den Fall, daß X die Gruppe -NR³-CO- darstellt, R³ gemeinsam mit den Stickstoffatom und einem C-Atom der Gruppe A einen heterocyclischen Ring mit 4 bis 6 C-Atomen aufspannt, oder R³ gemeinsam mit R², A und den beiden Stickstoffatomen einen heterocyclischen Ring mit 3 bis 5 C-Atomen aufspannt,
mit der Maßgabe, daß R₁ und R₂ nicht gleichzeitig Wasserstoff bedeuten, wenn X die Gruppe -NH-CO- oder -CO-NR₃- ist,
sowie deren optisch aktive Formen und physiologisch verträgliche Salze.

7. Nitroxyalkylamine ausgewählt aus der Gruppe der folgenden Verbindungen:
2-Amino-N-(1-nitroxy-2-methyl-prop-2-yl)-essigsäureamid,
4-Amino-N-(2-nitroxy-ethyl)-buttersäureamid,
cis-N-[2-(1-Pyrrolidinyl)-ethyl]-4-nitroxy-cyclohexancarbonsäureamid,
trans-2-Amino-2-methyl-N-(4-nitroxy-cyclohexyl)-propionsäureamid,
trans-N-(4-Nitroxy-cyclohexyl)-harnstoff,
sowie deren pharmakologisch verträgliche Salze, insbesondere mit organischen Säuren wie beispielsweise Oxalsäure, Maleinsäure, Fumarsäure oder Cyclohexylaminsulfonsäure.

8. Verfahren zur Herstellung von Nitroxyalkylamin-Derivaten der allgemeinen Formel I gemäß Anspruch 6 oder 7,
dadurch gekennzeichnet, daß man
a) eine Verbindung der allgemeinen Formel II 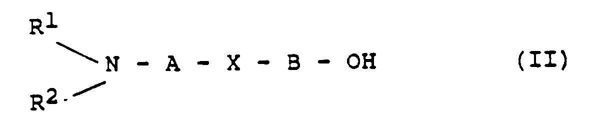 in der R¹, R², A, X und B die oben angegebenen Bedeutungen haben, einer Nitratester-Bildungsreaktion unterwirft, oder,
b) für den Fall, daß X eine -NR₃-CO-Gruppe bedeutet, eine Verbindung der allgemeinen Formel III 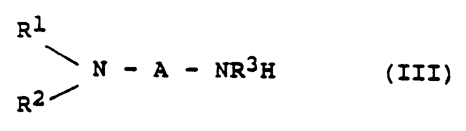 mit einer Verbindung der allgemeinen Formel IV
Z - CO - B - ONO₂ (IV)
wobei R¹, R², A, R³ und B die oben angegebenen Bedeutungen haben und Z eine nucleofuge Gruppe bedeutet, umsetzt,
c) für den Fall, daß X eine -CO-NR₃-Gruppe darstellt, eine Verbindung der allgemeinen Formel V
HNR³ - B - ONO₂ (V)
mit einer Verbindung der allgemeinen Formel VI 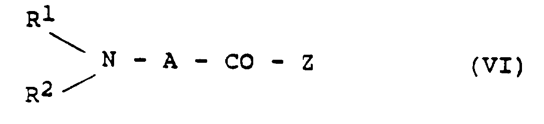 wobei R¹, R², A, R³, B und Z die oben angebenen Bedeutungen haben, umsetzt, oder
d) eine Verbindung der allgemeinen Formel VII
R² - NH - A - X - B - ONO₂ (VII)
mit Isocyanaten R¹-N=C=O oder Halogenameisensäure-Derivaten R¹-NH-CO-Hal umsetzt, wobei R¹, R², A, X und B die oben angegebenen Bedeutungen haben und Hal Chlor oder Brom bedeutet,
und gegebenenfalls anschließend die so erhaltenen Verbindungen in die optisch aktiven Formen oder physiologisch verträglichen Salze überführt.

9. Arzneimittel enthaltend mindestens eine Verbindung der Formel I gemäß Anspruch 6 oder 7 neben pharmazeutisch üblichen Träger- oder Hilfsstoffen.

10. Arzneimittel gemäß einem der Ansprüche 1 - 5 oder 9 zur Behandlung von Herz- und Kreislauferkrankungen.

11. Verwendung von Verbindungen der Formel I gemäß einem der Ansprüche 1 - 7 zur Herstellung von Arzneimitteln zur Behandlung von Herz- und Kreislauferkrankungen.

12. Verwendung von Verbindungen der allgemeinen Formel II 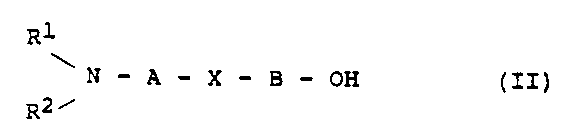 in der R¹, R², A, X und B die in den Ansprüchen 1 - 5 angegebenen Bedeutungen haben, zur Herstellung von Nitroxyalkylamin-Derivaten der allgemeinen Formel I gemäß einem der Ansprüche 1 - 6.

13. Verfahren zur Herstellung von Arzneimitteln enthaltend mindestens eine Verbindung der Formel I gemäß den Ansprüchen 1- 6, dadurch gekennzeichnet, daß man Verbindungen der Formel I mit pharmazeutisch üblichen Träger- oder Hilfsstoffen vermischt und zu pharmazeutischen Darreichungsformen verarbeitet.

## Claims

1. Medicament containing at least one nitroxyalkylamine derivative of the general formula I 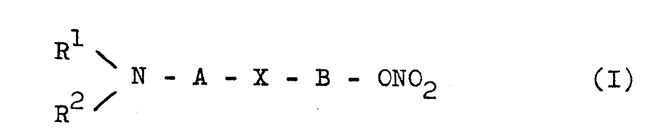 in which R¹ signifies hydrogen, a straight-chained or branched, saturated or unsaturated C₁-C₈-alkyl group, a C₃-C₈-cycloalkyl or C₈-cycloalkenyl group, an aminocarbonyl, C₁-C₆-alkylaminocarbonyl or di-C₁-C₆-alkylaminocarbonyl group, R² signifies hydrogen, a straight-chained or branched, saturated or unsaturated C₁-C₆-alkyl group or a C₃-C₈-cycloalkyl or C₈-cycloalkenyl group or R², together with R¹ and the nitrogen atom to which they are attached, form a heteroaliphatic ring with up to 6 C-atoms, wherein one or two C-atoms can also be replaced by nitrogen or oxygen atoms, A signifies a valency bond or a straight-chained or branched alkylene chain of 1 - 8 C-atoms, whereby a -CH₂- group can be replaced by a cycloalkylene group of 3 - 7 C-atoms, B signifies a straight-chained or branched alkylene chain of 1 - 8 C-atoms, whereby a -CH₂- group can be replaced by a cycloalkylene group of 3 - 7 C-atoms, X signifies an -NR³-CO- group of -CO-NR³- group, whereby R³ signifies hydrogen or a saturated or unsaturated alkyl group of 1 - 6 C-atoms or, for the case that X represents the group -NR³-CO-, R³, together with the nitrogen atom and a C-atom of the group A, bridge a heterocyclic ring with 4 to 6 C-atoms or R³, together with R², A and the two nitrogen atoms, bridge a heterocyclic ring containing 3 to 5 C-atoms; their optically-active forms or physiologically compatible salts, as well as pharmaceutically usual carrier and adjuvant materials.

2. Medicament containing at least one nitroxyalkylamine derivative of the formula I according to claim 1, in which R¹ signifies a hydrogen atom, a straight-chained or branched C₁-C₈-alkyl group, an aminocarbonyl or C₁-C₆-alkylaminocarbonyl group, R² a hydrogen atom, a straight-chained or branched C₁-C₆-alkyl group or R¹ and R², together with the nitrogen atom to which they are attached, form an aziridine, azetidine, pyrrolidine, piperidine, perhydroazepine, piperazine or morpholine ring.

3. Medicament containing at least one nitroxyalkylamine derivative of the formula I according to claim 1 or 2, in which A represents a valency bond or a methylmethylene, 1-methylethylene, dimethylmethylene, 1,1-dimethylethylene, 1,1-dimethyltrimethylene, 1-methyl-trimethylene, 1,1-dimethylmethylene, 1-ethylmethylene, 1,1-dimethyltetramethylene, 1,2-dimethyltrimethylene, 2,2-dimethyltrimethylene, 3,3-dimethyltrimethylene, 1,1-dimethylpentamethylene, 1,1-dimethylhexamethylene or 1-methyltetramethylene.

4. Medicament containing at least one nitroxyalkylamine derivative according to one of claims 1 - 3, in which B signifies ethylene, 1-methylethylene, 2-methylethylene, trimethylene, 1-methyltrimethylene, 3-methyltrimethylene, tetramethylene, pentamethylene, 1-methyltetramethylene, 1-ethyltrimethylene, hexamethylene, 1-methylpentamethylene, 1-ethyltetramethylene, 1-n-propyltrimethylene, 1,1-dimethylethylene, dimethylmethylene, 1,1-dimethyltrimethylene, 2,2-dimethyltrimethylene, 2,2-dimethyltetramethylene or 1,1-dimethylhexamethylene or a -CH₂- group of the chain B is replaced by the cyclopentylene group, the 1,2-, 1,3- or 1,4-cyclohexylene group, the methylene-1,2-cyclohexylene group, the methylene-1,3-cyclohexylene group or the methylene-1,4-cyclohexylene group, whereby the configuration of the cycloalkylene group can, in each case, be cis or trans.

5. Medicament containing at least one nitroxyalkylamine derivative of the general formula I according to one of claims 1 - 3, in which R³ is a hydrogen atom, a C₁-C₆-alkyl, C₂-C₆-alkenyl group or R³, together with the nitrogen atom and a C-atom of the group A, forms a pyrrolidine or piperidine ring or R³, together with R², A and the two nitrogen atoms, forms a piperazine ring.

6. Nitroxyalkylamine derivatives of the general formula I 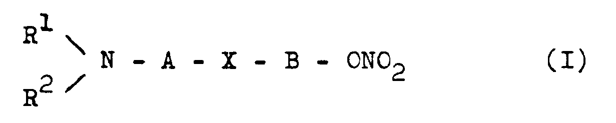 in which R¹ signifies hydrogen, a straight-chained or branched, saturated or unsaturated C₁-C₈-alkyl group, a C₃-C₈-cycloalkyl or C₈-cycloalkenyl group, an aminocarbonyl, C₁-C₆-alkylaminocarbonyl or di-C₁-C₆-alkylaminocarbonyl group, R² signifies hydrogen, a straight-chained or branched, saturated or unsaturated C₁-C₆-alkyl group or a C₃-C₈-cycloalkyl or C₈-cycloalkenyl group or R², together with R¹ and the nitrogen atom to which they are bound, form a heteroaliphatic ring with up to 6 C-atoms, wherein one or two C-atoms can also be replaced by nitrogen or oxygen atoms, A signifies a valency bond or a straight-chained or branched alkylene chain of 1 - 8 C-atoms, whereby a -CH₂- group can be replaced by a cycloalkylene group of 3 - 7 C-atoms, B signifies a straight-chained or branched alkylene chain of 1 - 8 C-atoms, whereby a -CH₂- group can be replaced by a cycloalkylene group of 3 - 7 C-atoms, X signifies an -NR³-CO- group or -CO-NR³- group, whereby R³ signifies hydrogen or a saturated or unsaturated alkyl group of 1 - 6 C-atoms or, for the case that X represents the group -NR³-CO-, R³, together with the nitrogen atom and a C-atom of the group A, bridge a heterocyclic ring with 4 to 6 C-atoms or R³, together with R², A and the two nitrogen atoms, bridge a heterocyclic ring with 3 to 5 C-atoms, with the proviso that R¹ and R² do not simultaneously represent hydrogen when X is the group -NH-CO- or -CO-NR³, as well as their optically-active forms and physiologically compatible salts.

7. Nitroxyalkylamines selected from the group of the following compounds:
2-amino-N-(1-nitroxy-2-methylprop-2-yl)-acetic acid amide,
4-amino-N-(2-nitroxyethyl)-butyric acid amide,
cis-N-[2-(1-pyrrolidinyl)-ethyl]-4-nitroxycyclohexane-carboxylic acid amide,
trans-2-amino-2-methyl-N-(4-nitroxycyclohexyl)-propionic acid amide,
trans-N-(4-nitroxycyclohexyl)-urea,
as well as their pharmacologically compatible salts, especially with organic acids, such as for example oxalic acid, maleic acid, fumaric acid or cyclohexylaminesulphonic acid.

8. Process for the preparation of nitroxyalkylamine derivatives of the general formula I according to claim 6 or 7, characterised in that one
a) subjects a compound of the general formula II 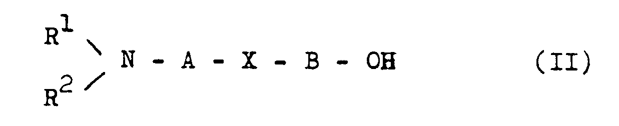 in which R¹, R², A, X and B have the above-given meanings, to a nitrate ester forming reaction; or
b) for the case that X signifies an -NR³-CO- group, reacts a compound of the general formula III 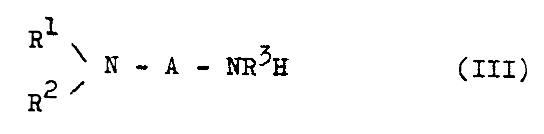 with a compound of the general formula IV
whereby R¹, R², A, R³ and B have the above-given meanings and Z signifies a nucleofuge group;
c)for the case that X represents a -CO-NR³- group, reacts a compound of the general formula V
HNR³ - B - ONO₂ (V)
with a compound of the general formula VI 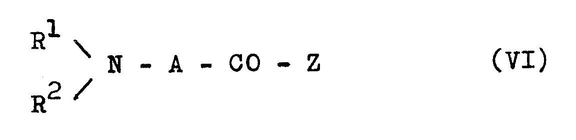 whereby R¹, R², A, R³, B and Z have the above-given meanings; or
d) reacts a compound of the general formula VII
R² - NH - A - X - B - ONO₂ (VII)
with isocyanates R¹-N=C=O or haloformic acid derivatives R¹-NH-CO-Hal, whereby R¹, R², A, X and B have the above-given meanings and Hal signifies chlorine or bromine, and possibly subsequently converts the so-obtained compounds into the optically-active forms or physiologically compatible salts.

9. Medicaments containing at least one compound of the formula I according to claim 6 or 7, besides pharmaceutically usual carrier or adjuvant materials.

10. Medicaments according to one of claims 1 - 5 or 9 for the treatment of heart and circulatory diseases.

11. Use of compounds of the formula I according to one of claims 1 - 7 for the production of medicaments for the treatment of heart and circulatory diseases.

12. Use of compounds of the general formula II 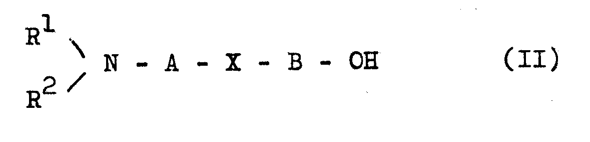 in which R¹, R², A, X and B have the meanings given in claims 1 - 5, for the preparation of nitroxyalkylamine derivatives of the general formula I according to one of claims 1 - 6.

13. Process for the preparation of medicaments containing at least one compound of the formula I according to claims 1 - 6, characterised in that one mixes compounds of the formula I with pharmaceutically usual carrier and adjuvant materials and works up to pharmaceutical forms of administration.

## Revendications

1. Médicaments contenant au moins un dérivé de nitroxyalkylamine de formule générale I 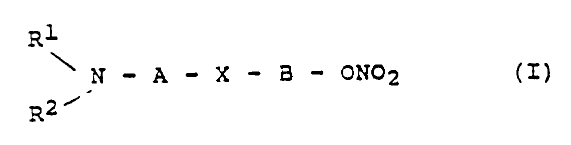 dans laquelle
R¹ représente un atome d'hydrogène, un groupe alkyle en C₁-C₈ à chaîne droite ou ramifiée, saturé ou insaturé, un groupe cycloalkyle en C₃-C₈ ou un groupe cycloalcényle en C₈, un groupe aminocarbonyle, un groupe alkyl(C₁-C₆)aminocarbonyle ou un groupe dialkyl(C₁-C₆)aminocarbonyle,
R² représente un atome d'hydrogène, un groupe alkyle en C₁-C₆ à chaîne droite ou ramifiée, saturé ou insaturé, ou un groupe cycloalkyle en C₃-C₈ ou un groupe cycloalcényle en C₈, ou R² forme, avec R¹ et l'atome d'azote auquel ils sont liés, un cycle hétéroaliphatique comportant jusqu'à 6 atomes de C, un ou deux atomes de C pouvant être également remplacés par des atomes d'azote ou d'oxygène,
A représente une valence libre ou une chaîne alkylène droite ou ramifiée comportant 1-8 atomes de C, un groupe -CH₂- pouvant être remplacé par un groupe cycloalkylène comportant 3-7 atomes de C,
B représente une chaîne alkylène droite ou ramifiée comportant 1-8 atomes de C, dans lequel un groupe -CH₂- peut être remplacé par un groupe cycloalkylène comportant 3-7 atomes de C,
X représente un groupe -NR³-CO ou -CO-NR³,
dans lequel
R³ représente un atome d'hydrogène ou un groupe alkyle à chaîne saturée ou insaturée, ayant 1-6 atomes de C, ou dans le cas où X représente le groupe -NR³-CO, R³ forme, conjointement avec l'atome d'azote et un atome de C du groupe A, un noyau hétérocyclique comportant 4 à 6 atomes de C, ou R³ forme, conjointement avec R², A et les deux atomes d'azote, un noyau hétérocyclique comportant 3 à 5 atomes de C,
ou leurs formes optiquement actives ou leurs sels physiologiquement acceptables, ainsi que des véhicules et adjuvants pharmaceutiques usuels.

2. Médicaments contenant au moins un dérivé de nitroxyalkylamine de formule I selon la revendication 1, dans laquelle
R¹ représente un atome d'hydrogène, un groupe alkyle en C₁-C₈ à chaîne droite ou ramifiée, un groupe aminocarbonyle ou un groupe alkyl(C₁-C₆)aminocarbonyle,
R2 représente un atome d'hydrogène, un groupe alkyle en C₁-C₆ à chaîne droite ou ramifiée,
ou R¹ et R² forment, conjointement avec l'atome d'azote auquel ils sont liés, un noyau aziridine, azétidine, pyrrolidine, pipéridine, perhydroazépine, pipérazine ou morpholine.

3. Médicaments contenant au moins un dérivé de nitroxyalkylamine de formule I, selon la revendication 1 ou 2, dans laquelle
A représente une valence libre ou un groupe méthylméthylène, 1-méthyléthylène, diméthylméthylène, 1,1-diméthyléthylène, 1,1-diméthyl-triméthylène, 1-méthyl-triméthylène, 1,1-diméthyl-méthylène, 1-éthylméthylène, 1,1-diméthyl-tétraméthylène, 1,2-diméthyl-triméthylène, 2,2-diméthyl-triméthylène, 3,3-diméthyl-triméthylène, 1,1-di-méthyl-pentaméthylène, 1,1-diméthyl-hexaméthylène ou 1-méthyl-tétraméthylène.

4. Médicaments contenant au moins un dérivé de nitroxyalkylamine selon l'une quelconque des revendications 1-3, dans lequel
B représente un groupe éthylène, 1-méthyl-éthylène, 2-méthyl-éthylène, triméthylène, 1-méthyl-triméthylène, 3-méthyl-triméthylène, tétraméthylène, pentaméthylène, 1-méthyl-tétra-méthylène, 1-éthyl-triméthylène, hexaméthylène, 1-méthyl-penta-méthylène, 1-éthyl-tétraméthylène, 1-n-propyl-triméthylène, 1,1-diméthyl-éthylène, diméthyl-méthylène, 1,1-di-méthyl-triméthylène, 2,2-diméthyl-triméthylène, 2,2-diméthyl-tétraméthylène ou 1,1-diméthyl-hexaméthylène, ou un groupe -CH₂- de la chaîne B est remplacé par le groupe cyclopentylène, le groupe 1,2-, 1,3- ou 1,4-cyclohexylène, le groupe méthylène-1,2-cyclohexylène, méthylène-1,3-cyclohexylène ou le groupe méthylène-1,4-cyclohexylène, la configuration du groupe cycloalkylène pouvant être chaque fois cis ou trans.

5. Médicaments contenant au moins un dérivé de nitroxyalkylamine de formule générale I suivant l'une quelconque des revendications 1-4,
dans laquelle
R³ représente un atome d'hydrogène, un groupe alkyle en C₁-C₆, un groupe alcényle en C₂-C₆, ou R³ forme, conjointement avec l'atome d'azote et un atome de C du groupe A, un noyau pyrrolidine ou pipéridine, ou R³ forme, conjointement avec R², A et les deux atomes de C, un noyau pipérazine.

6. Dérivé de nitroaxyalkylamine de formule générale I 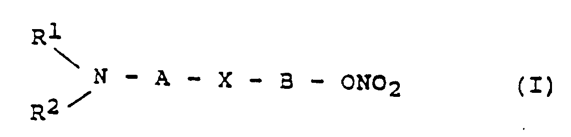 dans laquelle
R¹ représente un atome d'hydrogène, un groupe alkyle en C₁-C₈ à chaîne droite ou ramifiée, saturé ou insaturé, un groupe cycloalkyle en C₃-C₈ ou un groupe cycloalcényle en C₈, un groupe aminocarbonyle, un groupe alkyl(C₁-C₆)aminocarbonyle ou un groupe dialkyl(C₁-C₆)aminocarbonyle,
R² représente un atome d'hydrogène, un groupe alkyle en C₁-C₆ à chaîne droite ou ramifiée, saturé ou insaturé, ou un groupe cycloalkyle en C₃-C₈ ou un groupe cycloalcényle en C₈, ou R² forme, avec R¹ et l'atome d'azote auquel ils sont liés, un cycle hétéroaliphatique comportant jusqu'à 6 atomes de C, un ou deux atomes de C pouvant être également remplacés par des atomes d'azote ou d'oxygène,
A représente une valence libre ou une chaîne alkylène droite ou ramifiée comportant 1-8 atomes de C, un groupe -CH₂- pouvant être remplacé par un groupe cycloalkylène comportant 3-7 atomes de C,
B représente une chaîne alkylène droite ou ramifiée comportant 1-8 atomes de C, dans lequel un groupe -CH₂- peut être remplacé par un groupe cycloalkylène comportant 3-7 atomes de C,
X représente un groupe -NR³-CO ou -CO-NR³,
dans lequel
R³ représente un atome d'hydrogène ou un groupe alkyle à chaîne saturée ou insaturée, ayant 1-6 atomes de C, ou dans le cas où X représente le groupe -NR³-CO, R³ forme, conjointement avec l'atome d'azote et un atome de C du groupe A, un noyau hétérocyclique comportant 4 à 6 atomes de C, ou R³ forme, conjointement avec R², A et les deux atomes d'azote, un noyau hétérocyclique comportant 3 à 5 atomes de C,
étant entendu que R¹ et R² ne représentent pas simultanément un atome d'hyrogène lorsque X représente le groupe -NH-CO ou -CO-NR₃, ainsi que leurs formes optiquement actives et leurs sels physiologiquement acceptables.

7. Nitroxyalkylamine choisie dans le groupe constitué par les composés suivants :
amide de l'acide 2-amino-N-(1-nitroxy-2-méthyl-prop-2-yl)acétique,
amide de l'acide 4-amino-N-(2-nitroxy-éthyl)butyrique,
amide de l'acide cis-N-[2-(1-pyrrolidinyl)-éthyl]-4-nitroxy-cyclohexanecarboxylique,
amide de l'acide trans-2-amino-2-méthyl-N-(4-nitroxy-cyclohexyl)-propionique,
trans-N-(4-nitroxy-cyclohexyl)-urée,
ainsi que leurs sels pharmaceutiquement acceptables, en particulier ceux formés avec les acides organiques comme par exemple l'acide oxalique, l'acide maléique, l'acide fumarique ou l'acide cyclohexylaminosulfonique.

8. Procédé de préparation de dérivés de nitroxyalkylamine de formule générale I selon la revendication 6 ou 7 caractérisé en ce que
a) on soumet un composé de formule générale II 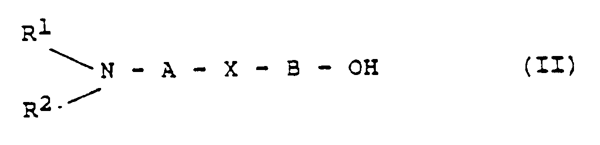 dans laquelle R¹, R², A, X et B ont les significations mentionnées ci-dessus, à une réaction de formation de nitrate-ester, ou
b) dans le cas où X représente un groupe -NR₃-CO, on fait réagir un composé de formule générale III 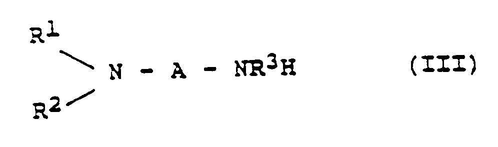 avec un composé de formule générale IV
Z-CO-B-ONO₂ (IV)
dans laquelle R¹, R², A, R³ et B ont les significations mentionnées ci-dessus et Z représente un groupe nucléofuge,
c) dans le cas où X représente un groupe -CO-NR₃, on fait réagir un composé de formule générale V
HNR³-B-ONO₂ (V)
avec un composé de formule générale VI 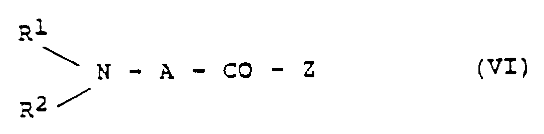 dans laquelle R¹, R², A, R³, B et Z ont les significations mentionnées ci-dessus, ou
d) on fait réagir un composé de formule générale VII
R²-NH-A-X-B-ONO₂ (VII)
avec des isocyanates R¹-N=C=O ou des dérivés d'acides halogénoformiques R¹-NH-CO-Hal, dans lesquels R¹, R², X et B ont les significations indiquées ci-dessus et Hal représente un atome de chlore ou de brome,
et ensuite, on transforme éventuellement les composés ainsi obtenus en leurs formes optiquement actives ou en leurs sels physiologiquement acceptables.

9. Médicaments contenant au moins un composé de formule I selon la revendication 6 ou 7, en plus de véhicules ou adjuvants pharmaceutiques usuels.

10. Médicaments selon l'une quelconque des revendications 1-5 ou 9, pour le traitement de maladies cardiovasculaires.

11. Utilisation des composés de formule I selon l'une quelconque des revendications 1-7, pour la préparation de médicaments pour le traitement de maladies cardiovasculaires.

12. Utilisation des composés de formule générale II 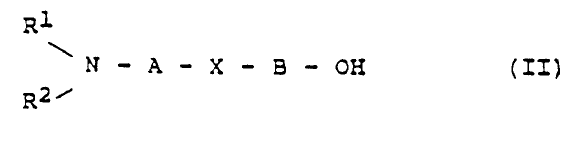 dans laquelle R¹, R², A, X et B ont les significations mentionnées dans les revendications 1 - 5, pour la préparation de dérivés de nitroxyalkylamine de formule générale I selon les revendications 1 - 6.

13. Procédé de préparation de médicaments contenant au moins un composé de formule I selon les revendications 1 - 6, caractérisé en ce qu'on mélange des composés de formule I avec des véhicules ou adjuvants pharmaceutiques usuels et les transforme en une forme galénique.
